# EUROPEAN PATENT SPECIFICATION

(11) **EP 0 963 370 B1**
(45) Date of publication and mention of the grant of the patent: **19.03.2003**
(21) Application number: 97952325.5
(22) Date of filing: 08.12.1997
(51) Int. Cl.: C07C 311/00, C07D 211/04, A61K 31/18, A61K 31/445

(54) **INTEGRIN ANTAGONISTS**
INTEGRIN ANTAGONISTEN
ANTAGONISTES DE L'INTEGRINE

(30) Priority: 09.12.1996 US 762117
(43) Date of publication of application: 15.12.1999
(73) Proprietor: ELI LILLY AND COMPANY, Indianapolis, Indiana 46285 (US); Millennium Pharmaceuticals, Inc., Cambridge, Massachusetts 02139 (US)
(72) Inventor: FISHER, Matthew, J., Carmel, IN 46033 (US); FRANCISKOVICH, Jeffry, Bernard, Indianapolis, IN 46254 (US); JAKUBOWSKI, Joseph, A., Indianapolis, IN 46208 (US); MASTERS, John, J., Indianapolis, IN 46236 (US); SCARBOROUGH, Robert, M., Belmont, CA 94002 (US); SMYTH, Mark, Foster City, CA 94404 (US); SU, Ting, Belmont, CA 94002 (US)
(74) Representative: Zimmermann & Partner
(86) International application number: US9722495
(87) International publication number: WO98025892

(56) References cited:
- WO-A-94/12181
- WO-A-97/06791
- WO-A-97/36859
- DE-A- 19 548 709

## Description

### Field of the Invention

This invention relates to novel α-sulfonamido and α-sulfinamido containing carboxylic acid compounds which are potent inhibitors of Arginyl-Glycyl-Aspartyl-(RGD)-dependent integrins. In another aspect, the present invention relates to these α-sutfonamido and α-sulfinamido containing carboxylic acids, their pharmaceutically-acceptable salts, and pharmaceutically-acceptable compositions thereof which are useful as potent inhibitors of integrin adhesive functions in mammals. In yet another aspect, the invention relates to methods for using these inhibitors as therapeutic agents for disease states in mammals characterized by aberrant cellular adhesive disorders that occur during thrombosis and restenosis.

### Background of the Invention

Cellular adhesion is believed to play an important role in both thrombus formation and cellular responses to vascular injury, as well as for normal hemostasis. Vascular injury and thrombosis are prevalent during the development and progression of vascular disease states. These include conditions such as atherosclerosis, acute myocardial infarction, chronic stable angina, unstable angina, transient ischemic attacks, stroke, peripheral vascular disease, arterial thrombosis, and conditions induced by interventional procedures such as restenosis following angioplasty.

Cellular adhesions can be characterized as either cell-cell adhesions or cell-matrix adhesions. Cells utilize a variety of cell surface adhesion receptors and adhesive proteins to facilitate these adhesive interactions. For cell-cell type adhesions, platelets play a major role in this type of adhesive interaction that occurs during acute thrombosis. Platelet aggregation, thrombus formation and consolidation of clots mediated by platelets are principally achieved by adhesive protein crosslinking of the platelet glycoprotein (GPIIb-IIIa) also referred to as α_{Πβ}β₃ which is found on the platelet surface. This heterodimeric adhesion receptor is one member of a large family of heterodimeric transmembrane glycoprotein receptors, called integrins (Hynes, R.O., "Integrins: Versatility, Modulation and Signaling in Cell Adhesion", Cell 69:11 (1992)).

Other integrins which may have important cell adhesion functions in thrombosis, hemostasis or in disease states characterized by vascular injury are the vitronectin receptors (αᵥβ₃ and αᵥβ₅) and the fibronectin receptor (α₅β₁). In particular, the vitronectin receptor, αᵥβ₃, has been postulated to play roles in cellular migration of smooth muscle cells following vascular injury that can ultimately lead to restenosis of the vessel (Yue, T.L., *et* *al*., "Osteopontin-Stimulated Vascular Smooth Muscle Cell Migration is Mediated by β₃ Integrin", Exp. Cell. Res. 214:459-464 (1994); Choi, E.T., *et al.,* "Inhibition of Neointimal Hyperplasia by Blocking αᵥβ₃ Integrin with a Small Peptide Antagonist GpenGRGDSPCA", J. Vasc. Surg. 19:125-134 (1994); Matsuno, H., "Inhibition of Integrin Function by A Cyclic RGD-Containing Peptide Prevents Neointima Formation", Circulation 90:2203-2206 (1994)). A number of the natural ligands of these integrins (e.g. α_{Πβ}β₃, αᵥβ₃, αᵥβ₅, and α₅β₁) such as fibrinogen, fibronectin, von Willebrand factor, thrombospondin, osteopontin, vitronectin and others, contain and utilize the tripeptide sequence, Arg-Gly-Asp (RGD) to bind to their respective integrins. Small synthetic peptides containing the RGD sequence have been shown to bind to these integrins and to compete for the binding of natural adhesive ligands (Rouslahti, E. and Pierschbacher, M.D., "New Perspectives in Cell Adhesion: RGD and Integrins", Science 238:491-497 (1987)). Peptides containing the RGD sequence or mimetic compounds have thus been the basis for the discovery of several potent and highly specific inhibitors of platelet α_{Πβ}β₃ which are useful as antithrombotic agents. This literature has been extensively reviewed. See Coller, B.S., "Blockade of Platelet GPIIb/IIIa Receptors as an Antithrombotic Strategy", Circulation 92:2373-2380 (1995); Cook, N.S., *et al*., "Platelet Glycoprotein IIb/IIIa Antagonists", Drugs of the Future 19:135-159 (1994); T. Weller., *et al*., "Fibrinogen Receptor Antagonists - A Novel Class of Promising Antithrombotics", Drugs of the Future 19:461 (1994); and Zablocki, J.A., *et al.,* "Fibrinogen Receptor Antagonists", Exp. Opin. Invest. Drugs. 3:437-448 (1994).

Highly specific inhibitors of αᵥβ₃ based on the RGD recognition sequence have also been recently described. Specifically, the cyclic peptide, cyclo[Arg-Gly-Asp-D-Phe-Val] is a very potent and specific inhibitor of the vitronectin receptor αᵥβ₃ (Pfaff, M., *et al*., "Selective Recognition of Cyclic RGD Peptides of NMR Defined Conformation by α_{Πβ}β₃, αᵥβ₃, and α₅β₁ Integrins", J. Biol. Chem. 269:20233-20238 (1994); Jonczyk, A., *et al*., European Patent Application 578083A2 (1994)).

The present invention describes the preparation of novel compounds which inhibit the adhesive function of various RGD-dependent integrins. More specifically, the novel compounds are non-specific inhibitors of the platelet integrin α_{Πβ}β₃ and the vitronectin receptor αᵥβ₃.

### Summary of the Invention

The present invention relates to novel α-sulfonamido and α-sulfinamido containing carboxylic acids or carboxylic esters, their pharmaceutically-acceptable stereoisomers, salts, hydrates, solvates and prodrug derivatives, and pharmaceutically-acceptable compositions thereof which have particular biological properties and are useful as potent antithrombotics and/or antirestenotic agents in mammals.

The present invention provides a compound of the formula: 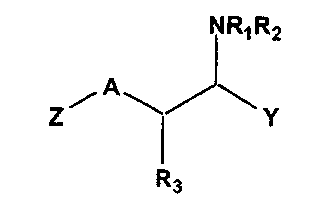 wherein:
Y is selected from the group consisting of -COOH, -PO₃H₂, -SO₃H and -COOR⁴; where R⁴ is selected from the group consisting of C₁₋₁₀alkyl, C₁₋₈alkylaryl, aryl-C₁₋₈alkyl, C₁₋₈alkyloxycarbonyloxy-C₁₋₈alkyl, aryloxycarbonyloxy-C₁₋₈alkyl, C₁₋₈alkyloxycarbonyloxyaryl, C₁₋₈alkylcarbonyloxy-C₁₋₈alkyl, arylcarbonyloxy-C₁₋₈alkyl and C₁₋₈alkylcarbonyloxyaryl;
A is selected from the group consisting of C₆₋₁₂alkyl, C₀₋₈alkyl-NR⁵CO-C₀₋₈alkyl, C₀₋₈alkyl-CO-NR⁵-C₀₋₈alkyl, C₀₋₈alkyl-O-C₀₋₈alkyl, C₀₋₈alkyl-NR⁵-CO-C₁₋₈alkyl-NR⁵-CO-C₀₋₈alkyl, C₀₋₈alkyl-NR⁵-CO-C₁₋₈alkyl-CO-NR⁵-C₀₋₈alkyl, C₀₋₈alkyl-CO-NR⁵-C₁₋₈alkyl-NR⁵-CO-C₀₋₈alkyl, C₀₋₈alkyl-CO-NR⁵-C₀₋₈alkyl-CO-NR⁵-C₀₋₈alkyl, C₀₋₈alkyl-CO-C₁₋₈alkyl-CO-NR⁵-C₀₋₈alkyl, C₀₋₈alkyl-CO-C₀₋₈alkyl-NR⁵-CO-C₀₋₈alkyl, C₀₋₈alkyl-O-C₂₋₈alkyl-NR⁵-CO-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₀₋₈alkyl, C₀₋₈alkyl-S(Oₙ)-C₀₋₈alkyl, C₀₋₈alkyl-S-C₂₋₈alkyl-NR⁵-CO-C₀₋₈alkyl, C₀₋₈alkyl-S(Oₙ)-C₂₋₈alkyl-NR⁵-CO-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-CO-NR⁵-C₂₋₈alkyl-S-C₀₋₈alkyl, C₀₋₈alkyl-CO-NR⁵-C₂₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CS-O-C₀₋₈alkyl, C₀₋₈alkyl-NR⁵-C₀₋₈alkyl-CO-NR⁵-C₀₋₈alkyl, C₀₋₈alkyl-NR⁵-C₀₋₈alkyl-CS-NR⁵-C₀₋₈alkyl, C₀₋₈alkyl-O-C₀₋₈alkyl-CO₂-C₀₋₈alkyl, C₀₋₈alkyl-O-C₀₋₈alkyl-CS-O-C₀₋₈alkyl; C₀₋₈alkyl-SiR⁷R⁸-C₀₋₈alkyl, C₀₋₈alkyl-SiR⁷R⁸-C₀₋₈alkyl-NR⁶-CO-C₀₋₈alkyl, and C₀₋₈alkyl-SiR⁷R⁸-C₀₋₈alkyl-CO-NR⁶-C₀₋₈alkyl; where R⁵, R⁶, R⁷ and R⁸ are independently selected from the group consisting of H and C₁₋₆alkyl; and where n=1 or 2;
Z is selected from the group consisting of -NH-C(NR⁹R¹⁰)=NR¹¹, -NH-C(R⁹)=NR¹¹,-C(NR⁹R¹⁰)=NR¹¹ and piperidinyl; where R⁹, R¹⁰ and R¹¹ are independently selected from the group consisting of H, C₁₋₆alkyl, aryl-C₁₋₃alkyl and aryl; or where two of the R⁹, R¹⁰ or R¹¹ substituents form a cyclic ring containing (CH₂)ₚ, where p=2-5;
R¹ is H;
R² is selected from the group consisting of -SOₘ-aryl, -SOₘ-C₁₋₁₀alkyl and -SOₘ-heteroaryl, where m=1-2;
R³ is selected from the group consisting of H, C₁₋₈alkyl, aryl, C₁₋₈alkylaryl and heteroaryl;
and all pharmaceutically-acceptable stereoisomers, salts, hydrates, solvates and prodrug derivatives thereof.

In certain aspects of this invention, compounds are provided which are useful as diagnostic reagents. In another aspect, the present invention includes pharmaceutical compositions comprising a pharmaceutically-effective amount of the compounds of this invention and a pharmaceutically-acceptable carrier. In yet another aspect, the present invention includes methods which comprise administering the compounds of the present invention and pharmaceutical compositions thereof for preventing or treating disease states characterized by thrombosis or vascular injury in mammals. Optionally, the methods of this invention comprise administering such pharmaceutical compositions in combination with an additional therapeutic agent such as an antithrombotic, a thrombolytic agent or an anticoagulant, or any combination thereof.

The preferred compounds also include their pharmaceutically-acceptable stereoisomers, hydrates, solvates, salts and prodrug derivatives.

### Detailed Description of the Invention

### Definitions

In accordance with the present invention and as used herein, the following terms are defined with the following meanings, unless explicitly stated otherwise.

The term "alkyl" refers to saturated and unsaturated aliphatic groups including straight-chain and branched-chain and cyclic groups, or any combination thereof, having the number of carbon atoms specified, or if no number is specified, having up to 12 carbon atoms. Cyclic alkyls typically comprise a monocyclic aliphatic ring having 3 to 12 carbon atoms and preferably 3 to 7 carbon atoms. The cyclic alkyls of this invention may include one or more nitrogen atoms. Preferably, "alkyl" refers to straight-chain and branched-chain groups; more preferably straight-chain groups.

The term "aryl" refers to an unsubstituted or substituted aromatic ring, substituted with one, two or three substituents selected from C₁₋₆alkoxy, C₁₋₆alkyl, C₁₋₆alkylamino, hydroxy, halogen, cyano, hydroxyl, mercapto, nitro, thioalkoxy, carboxaldehyde, carboxyl, carboalkoxy and carboxamide, including but not limited to carbocyclic aryl, heterocyclic aryl, and biaryl groups and the like, all of which may be optionally substituted. Preferred aryl groups include phenyl, halophenyl, C₁₋₆alkylphenyl, naphthyl, biphenyl, phenanthrenyl, naphthacenyl, and aromatic heterocyclics. The term "heteroaryl" as used herein refers to any aryl group, containing from one to four heteroatoms, selected from the group consisting of nitrogen, oxygen and sulfur.

The term "arylalkyl" refers to one, two, or three aryl groups appended to an alkyl group having the number of carbon atoms designated. Suitable arylalkyl groups include, but are not limited to, benzyl, picolyl, naphthylmethyl, phenethyl, benzhydryl, trityl, and the like, all of which may be optionally substituted. Similarly, the term "alkylaryl" refers to an alkyl group, having the number of carbon atoms designated, appended to one, two, or three aryl groups.

The terms "halo" or "halogen" as used herein refer to Cl, Br, F or I substituents.

The term "oxy" refers to an oxygen (O) atom. The terms "alkyloxy" and "aryloxy" thus refer to the respective groups positioned adjacent to an oxygen atom. The term "carbonyloxy" refers to -C(O)-O-.

The term "pharmaceutically-acceptable salts" includes salts of compounds derived from the combination of a compound of the present invention and an organic or inorganic acid. These compounds are useful in both free base and salt form. In practice, the use of the salt form amounts to use of the base form; both acid and base addition salts are within the scope of the present invention.

"Pharmaceutically-acceptable acid addition salt" refers to those salts which retain the biological effectiveness and properties of the free bases and which are not biologically or otherwise undesirable, formed with inorganic acids such as hydrochloric acid, hydrobromic acid, hyroiodic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

"Pharmaceutically-acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium and magnesium salts. Salts derived from pharmaceutically-acceptable organic nontoxic bases include salts of primary, secondary, and tertiary amines, substituted amines including naturally occurring substituted amines, cyclic amines and basic ion-exchange resins, such as isopropylamine, trimethylamine, diethylamine, triethylamine, tripropylamine, ethano,amine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, N-ethylpiperidine, polyamine resins and the like. Particularly preferred organic nontoxic bases are isopropylamine, diethylamine, ethanolamine, trimethamine, dicyclohexylamine, choline, and caffeine.

The term "prodrug derivatives" refers to compounds of the invention which have metabolically cleavable groups and become, by sovolysis or under physiological conditions, compounds of the invention which are pharmaceutically-active *in vivo.* Fro example, ester derivatives of compounds of this invention are often active *in vivo,* but may have only weak or no activity *in vitro.* Other derivatives of the compounds of this invention have activity in both their acid and acid derivative forms, but the acid derivative form often offers advantages of solubility, tissue compatibility, or delayed release in the mammalian organism. See Bundgard, H., "Design of Prodrugs", pp. 7-9, 21-24, Elsevier, Amsterdam, 1985. Prodrugs include acid derivatives well known to practitioners of the art, such as esters prepared by the reaction of the parent acid with a suitable alcohol, or amides prepared by reaction of the parent acid with an amine. Simple aliphatic or aromatic esters derived from acidic groups pendant on the compounds of this invention are preferred prodrug derivatives. In some cases, it is desirable to prepare double ester-type prodrugs such as (aclyoxy)alkyl esters or [(alkoxycarbonyl)oxy]alkyl esters.

"Biological property" for the purposes herein means an *in vivo* effector or antigenic function or activity that is directly or indirectly performed by a compound of this invention. Effector functions include receptor or ligand binding, any enzyme activity or enzyme modulatory activity, any carrier binding activity, any hormonal activity, any activity in promoting or inhibiting adhesion of cells to an extracellular matrix or cell surface molecules, or any structural role. Antigenic functions include possession of an epitope or antigenic site that is capable of reacting with antibodies raised against it.

In addition, the following are used in this application:
- "Boc": t-butoxycarbonyl
- "BOP": benzotriazol-1-yloxy-tris-(dimethylamino)phosphonium hexafluorophosphate
- "Cbz": benzyloxycarbonyl
- "DCC": N,N'-dicyclohexylcarbodiimide
- "DIEA": diisopropylethylamine
- "DMAP": 4-dimethylaminopyridine
- "DMF": N,N-dimethylformamide
- "HBTU": 2-(1-H-benzotriazol-1-yl)-1,1,3,3,-tetramethyluronium hexafluorophosphate
- "HF": hydrogen fluoride
- "HOBt": N-hydroxybenzotriazole
- "MeOH": methanol
- "Ph": phenyl
- "PhSO₂NH-": phenylsulfonamido
- "TFA": trifluoroacetic acid

In the compounds of this invention, carbon atoms bonded to four non-identical substituents are asymmetric. Accordingly, the compounds may exist as stereoisomers, including enantiomers and diastereomers. The compounds of this invention having one or more centers of asymmetry may exist as enantiomers or mixtures thereof (e.g. racemates). In addition, compounds that have two or more asymmetric centers can exist as diastereomers. The syntheses described herein may employ racemates, enantiomers or diastereomers as starting materials or intermediates. Diastereomeric products resulting from such syntheses may be separated by chromatographic or crystallization methods, or by other methods known in the art. Likewise, enantiomeric product mixtures may be separated using methods known in the art. See for example, Jacqes, Collet and Wilen "Enantiomers, Racemates and Resolutions" (Krieger Publishing Co., Malabar, FL 1991). Each of the asymmetric centers, when present in the compounds of this invention, may be in one of two configurations (R or S), and both are within the scope of the present invention. Some of the compounds may be designated either D or L, which is a less preferred indicator of the configuration of the compound, based on the compound of this invention having a configuration that is similar to known amino acids. In the processes described above, the final products may, in some cases, contain a small amount of the products having D or L-form residues; however, these products do not affect their therapeutic or diagnostic application.

In all of the compounds of the invention having one or more amide linkages (-CO-NH-), such amide linkages may optionally be replaced with another linkage which is an isostere such as -CH₂NH-, -CH₂S-, CH₂-O, CH₂CH₂, -CH=CH- (cis and trans), -COCH₂-, -CH(OH)CH₂-, -CH₂SO-, and CH₂SO₂. This replacement can be made by methods known in the art. The following references describe preparation of peptide analogs which include these alternative-linking moieties: Spatola, A.F., Vega Data (March 1983), Vol. 1, Issue 3, "Peptide Backbone Modifications" (general review); Spatola, A.F., in "Chemistry and Biochemistry of Amino Acids, Peptides and Proteins," B. Weinstein, eds., Marcel Dekker, New York, p. 267 (1983) (general review); Morley, J.S., Trends Pharm Sci pp. 463-468 (1980) (general review); Hudson, D., *et al.,* Int J Peat Prot Res 14:177-185 (1979) (-CH₂NH-, -CH₂CH₂-); Spatola, A.F., *et al.,* Life Sci 38:1243-1249(1986) (-CH₂-S); Hann, M.M., J Chem Soc Perkin Trans I pp. 307-314 (1982) (-CH=CH-, cis and trans); Almquist, R.G., *et al*., J Med Chem 23:1392-1398 (1980) (-COCH₂-); Jennings-White, C., *et al.,* Tetrahedron Lett 23:2533(1982) (-COCH₂-); Szelke, M., *et al.*, European Application EP 45665; CA 97:39405 (1982) (-CH(OH)CH₂-); Holladay, M.W., *et al.,* Tetrahedron Lett 24:4401-4404 (1983) (-C(OH)CH₂-); and Hruby, V.J., Life Sci 31:189-199 (1982) (-CH₂-S-).

### Preferred Embodiments

In preferred embodiments, the present invention provides compounds of the formula: 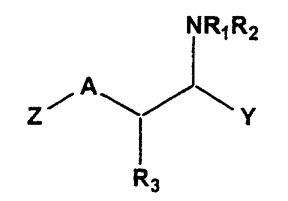 wherein:
Y is selected from the group consisting of -COOH, -PO₃H₂, -SO₃H and -COOR⁴; where R⁴ is selected from the group consisting of C₁₋₁₀alkyl, C₁₋₈alkylaryl, aryl-C₁₋₈alkyl, C₁₋₈alkyloxycarbonyloxy-C₁₋₈alkyl, aryloxycarbonyloxy-C₁₋₈alkyl, C₁₋₈alkyloxycarbonyloxyaryl, C₁₋₈alkylcarbonyloxy-C₁₋₈alkyl, arylcarbonyloxy-C₁₋₈alkyl and C₁₋₈alkylcarbonyloxyaryl;
A is selected from the group consisting of C₆₋₁₂alkyl, C₀₋₈alkyl-NR⁵-CO-C₀₋₈alkyl, C₀₋₈alkyl-CO-NR⁵-C₀₋₈alkyl, C₀₋₈alkyl-O-C₀₋₈alkyl, C₀₋₈alkyl-NR⁵-CO-C₁₋₈alkyl-NR⁵-CO-C₀-₈alkyl, C₀₋₈alkyl-NR⁵-CO-C₁₋₈alkyl-CO-NR⁵-C₀₋₈alkyl, C₀₋₈alkyl-CO-NR⁵-C₁₋₈alkyl-NR⁵-CO-C₀₋₈alkyl, C₀₋₈alkyl-CO-NR⁵-C₀₋₈alkyl-CO-NR⁵-C₀₋₈alkyl, C₀₋₈alkyl-CO-C₁₋₈alkyl-CO-NR⁵-C₀₋₈alkyl, C₀₋₈alkyl-CO-C₀₋₈alkyl-NR⁵-CO-C₀₋₈alkyl, C₀₋₈alkyl-O-C₂₋₈alkyl-NR⁵-CO-C₀₋₈alkyl, C₀-₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₀₋₈alkyl, C₀₋₈alkyl-S(Oₙ)-C₀₋₈alkyl, C₀₋₈alkyl-S-C₂₋₈alkyl-NR⁵-CO-C₀₋₈alkyl, C₀₋₈alkyl-S(Oₙ)-C₂₋₈alkyl-NR⁵-CO-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S (Oₙ)-C₀₋₈alkyl, C₀₋₈alkyl-CO-NR⁵-C₂₋₈alkyl-S-C₀₋₈alkyl, C₀₋₈alkyl-CO-NR⁵-C₂₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CS-O-C₀₋₈alkyl, C₀₋₈alkyl-NR⁵-C₀₋₈alkyl-CO-NR⁵-C₀₋₈alkyl, C₀₋₈alkyl-NR⁵-C₀₋₈alkyl-CS-NR⁵-C₀₋₈alkyl, C₀₋₈alkyl-O-C₀₋₈alkyl-CO₂-C₀₋₈alkyl, C₀₋₈alkyl-O-C₀₋₈alkyl-CS-O-C₀₋₈alkyl; C₀₋₈alkyl-SiR⁷R⁸-C₀₋₈alkyl, C₀₋₈alkyl-SiR⁷R⁸-C₀₋₈alkyl-NR⁶-CO-C₀₋₈alkyl, and C₀₋₈alkyl-SiR⁷R⁸-C₀₋₈alkyl-CO-NR⁶C₀₋₈alkyl; where R⁵, R⁶, R⁷ and R⁸ are independently selected from the group consisting of H and C₁₋₆alkyl; and where n=1 or 2;
Z is selected from the group consisting of -NH-C(NR⁹R¹⁰)=NR¹¹, -NH-C(R⁹)=NR¹¹,-C(NR⁹R¹⁰)=NR¹¹ and piperidinyl; where R⁹, R¹⁰ and R¹¹ are independently selected from the group consisting of H, C₁₋₆alkyl, aryl-C₁₋₃alkyl and aryl; or where two of the R⁹, R¹⁰ or R¹¹ substituents form a cyclic ring containing (CH₂)ₚ, where p=2-5;
R¹ is H;
R² is selected from the group consisting of -SOₘ-aryl, -SOₘ-C₁₋₁₀alkyl and -SOₘ-heteroaryl, where m=1-2;
R³ is selected from the group consisting of H, C₁₋₈alkyl, aryl, C₁₋₈alkylaryl and heteroaryl;
and all pharmaceutically-acceptable stereoisomers, salts, hydrates, solvates and prodrug derivatives thereof.

It is understood that the "A" substituents can be incorporated in the compounds of the invention in the order written above or in the reverse order. For example, a suitable "A" substituent is C₀₋₈alkyl-O-C₀₋₈alkyl-CO-NR⁵-C₀₋₈alkyl. It is understood that the "Z" substituent can be positioned to the right or to the left of this sequence.

Preferred "Y" substituents are -COOH and -COOR⁴, more preferably -COOH. R⁴ is preferably C₁₋₁₀alkyl.

Preferred "A" substituents are selected from the group consisting of C₀₋₈alkyl-NR⁵-CO-C₀₋₈alkyl, C₀₋₈alkyl-CO-NR⁵-C₀₋₈alkyl, C₀₋₈alkyl-NR⁵-CO-C₁₋₈alkyl-NR⁵-CO-C₀₋₈alkyl, C₀₋₈alkyl-NR⁵-CO-C₁₋₈alkyl-CO-NR⁵-C₀₋₈alkyl, C₀₋₈alkyl-CO-NR⁵-C₁₋₈alkyl-NR⁵-CO-C₀₋₈alkyl, C₀₋₈alkyl-CO-NR⁵-C₀₋₈alkyl-CO-NR⁵-C₀₋₈alkyl, C₀₋₈alkyl-CO-C₁₋₈alkyl-CO-NR⁵-C₀₋₈alkyl, C₀₋₈alkyl-O-C₂₋₈alkyl-NR⁵-CO-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₂₋₈alkyl-NR⁵-CO-C₀₋₈alkyl, C₀₋₈alkyl-S(Oₙ)-C₂₋₈alkyl-NR⁵-CO-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-CO-NR⁵-C₂₋₈alkyl-S-C₀₋₈alkyl, C₀₋₈alkyl-CO-NR⁵-C₂₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CS-O-C₀₋₈alkyl, C₀₋₈alkyl-NR⁵-C₀₋₈alkyl-CO-NR⁵-C₀₋₈alkyl, C₀₋₈alkyl-NR⁵-C₀₋₈alkyl-CS-NR⁵-C₀₋₈alkyl, C₀₋₈alkyl-O-C₀₋₈alkyl-CO₂-C₀₋₈alkyl, C₀₋₈alkyl-O-C₀₋₈alkyl-CS-O-C₀₋₈alkyl; C₀₋₈alkyl-SiR⁷R⁸-C₀₋₈alkyl, C₀₋₈alkyl-SiR⁷R⁸-C₀₋₈alkyl-NR⁶-CO-C₀₋₈alkyl and C₀₋₈alkyl-SiR⁷R⁸-C₀₋₈alkyl-CO-NR⁶-C₀₋₈alkyl.

More preferred "A" substituents are selected from the group consisting of C₀₋₈alkyl-CO-NR⁵-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-NR⁵C₀₋₈alkyl-CO₂-C₀₋₈alkyl, C₀₋₈alkyl-NR⁵-C₀₋₈alkyl-CO-NR⁵-C₀₋₈alkyl, C₀₋₈alkyl-O-C₀₋₈alkyl-CO₂-C₀₋₈alkyl.

Preferably, "Z" is -NH-C(NR⁹R¹⁰)=NR¹¹. Preferably R⁹, R¹⁰ and R¹¹ are independently selected from the group consisting of H and C₁₋₆alkyl. More preferably, R⁹, R¹⁰ and R¹¹ are H.

Preferred R² substituents are -SO₂-aryl and -SO₂-C₁₋₁₀alkyl. More preferably, R² is-SO₂-aryl.

Preferred R³ substituents are H and C₁₋₈alkyl. More preferably, R³ is H.

Preferred compounds and subgroups of compounds may be selected from any combination of the formulas presented in this specification with one or more of the preferred groupings of substituents at a particular location.

Other preferred compounds of the present invention are shown but not limited to the following list of compounds which have the structure:

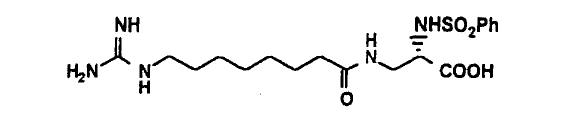

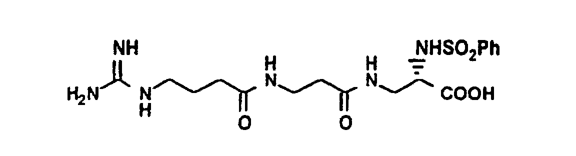

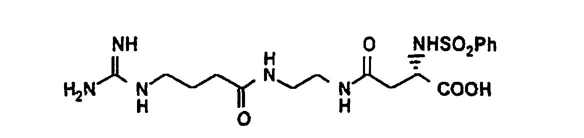

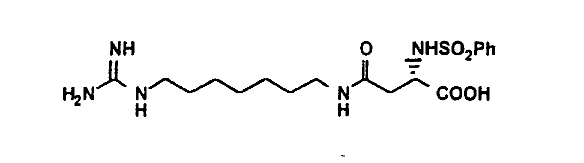

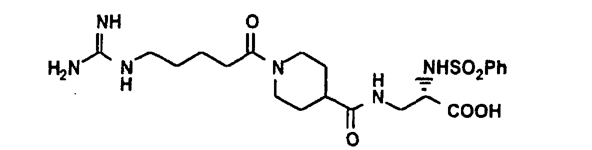

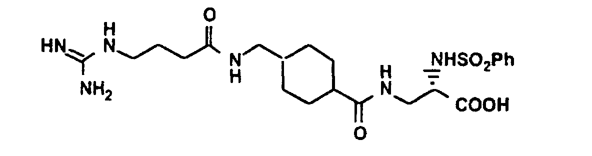

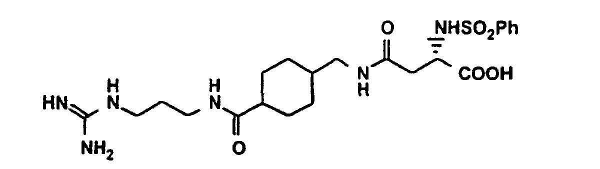

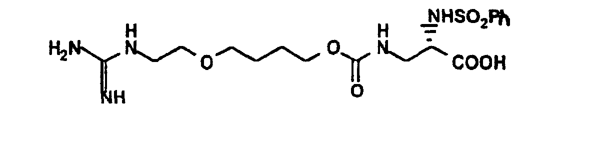

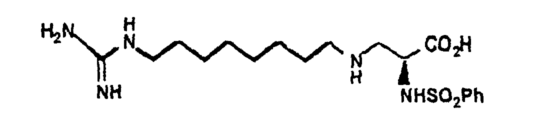

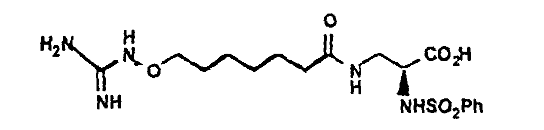

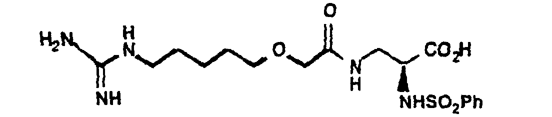

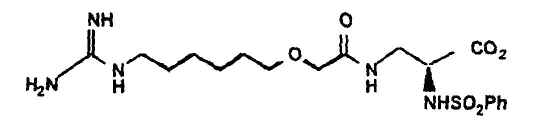

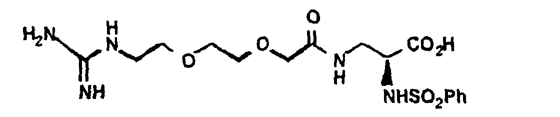

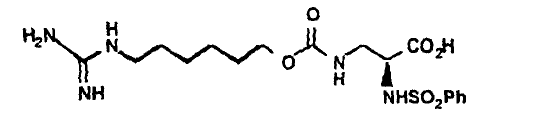

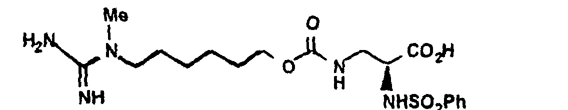

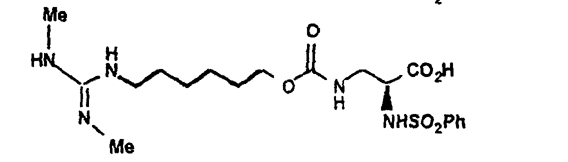

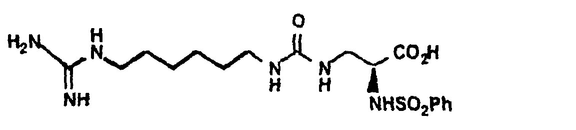

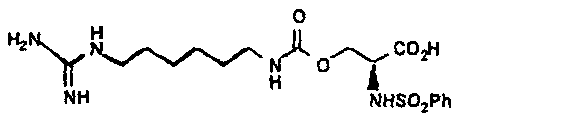

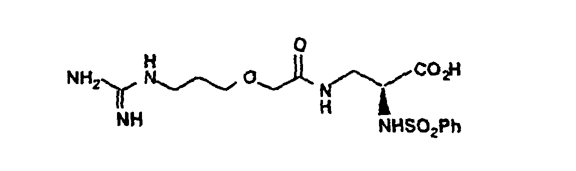

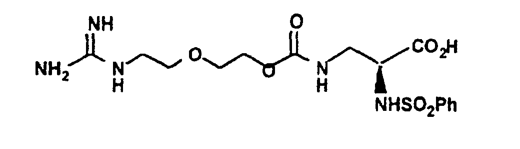

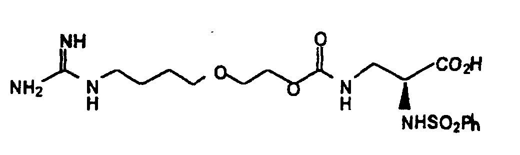

This invention also encompasses prodrug derivatives of the compounds contained herein. The term "prodrug" refers to a pharmacologically-inactive derivative of a parent drug molecule that requires biotransformation, either spontaneous or enzymatic, within the organism to release the active drug. Prodrugs are variations or derivatives of the compounds of this invention which have metabolically cleavable groups and become, by solvolysis under physiological conditions, or by enzymatic degradation, compounds which are pharmaceutically active *in vivo.* Prodrug compounds of this invention may be called single, double, triple etc., depending on the number of biotransformation steps required to release the active drug within the organism, and indicating the number of functionalities present in a precursor-type form. Prodrug forms often offer advantages of solubility, tissue compatibility, or delayed release in the mammalian organism (see, Bundgard, H., Design of Prodrugs, pp. 7-9, 21-24, Elsevier, Amsterdam 1985 and Silverman, R.B., The Organic Chemistry of Drug Design and Drug Action, pp. 352-401, Academic Press, San Diego, CA, 1992). Prodrugs commonly known in the art include acid derivatives well known to practitioners of the art, such as, for example, esters prepared by reaction of the parent acids with a suitable alcohol, or amides prepared by reaction of the parent acid compound with an amine, or basic groups reacted to form an acylated base derivative. Moreover, the prodrug derivatives of this invention may be combined with other features herein taught to enhance bioavailability.

### Preparation of Compounds

The compounds of the present invention may be synthesized by either solid or liquid phase methods described and referenced in standard textbooks, or by a combination of both methods. These methods are well known in the art. See, Bodanszky, M., in "The Principles of Peptide Synthesis", Hafner, K., Rees, C.W., Trost, B.M., Lehn, J.-M., Schleyer, P. v-R., Zahradnik, R., Eds., Springer-Verlag, Berlin, 1984. Starting materials are commercially available reagents and reactions are carried out in standard laboratory glassware and reaction vessels under reaction conditions of standard temperature and pressure, except where otherwise indicated.

The starting materials used in any of these methods are commercially available from chemical vendors such as Aldrich, Sigma, Nova Biochemicals, Bachem Biosciences, and the like, or may be readily synthesized by known procedures.

During the synthesis of these compounds, the functional groups of the amino acid derivatives used in these methods are protected by blocking groups to prevent cross reaction during the coupling procedure. Examples of suitable blocking groups and their use are described in "The Peptides: Analysis, Synthesis, Biology", Academic Press, Vol. 3 (Gross, E. & Meienhofer, J., Eds., 1981) and Vol. 9 (, S. &., Eds., 1987), the disclosures of which are incorporated herein by reference.

Nine exemplary synthesis schemes are outlined below, and the specific syntheses are described in the Examples. The reaction products are isolated and purified by conventional methods, typically by solvent extraction into a compatible solvent. The products may be further purified by column chromatography or other appropriate methods.

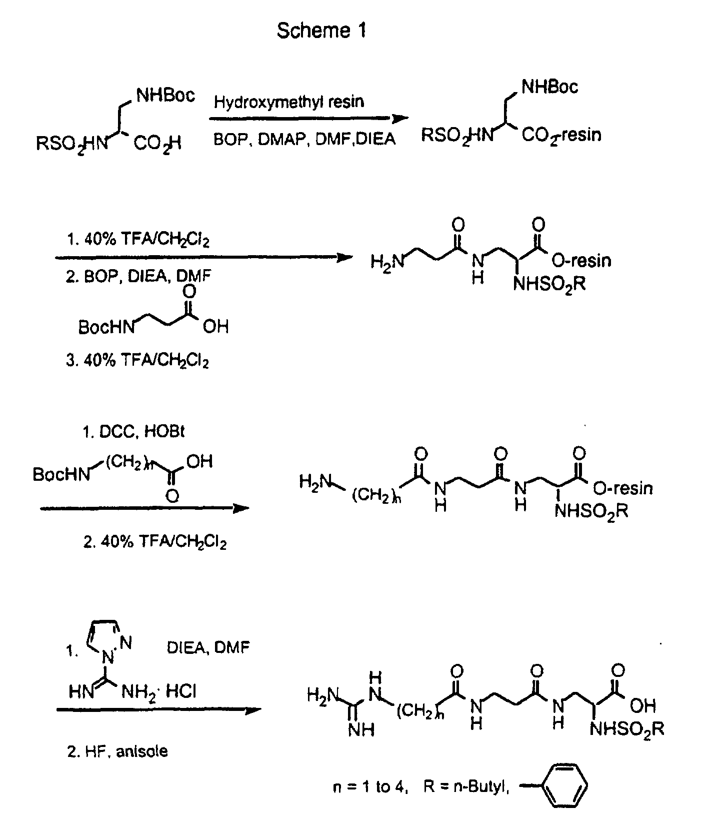

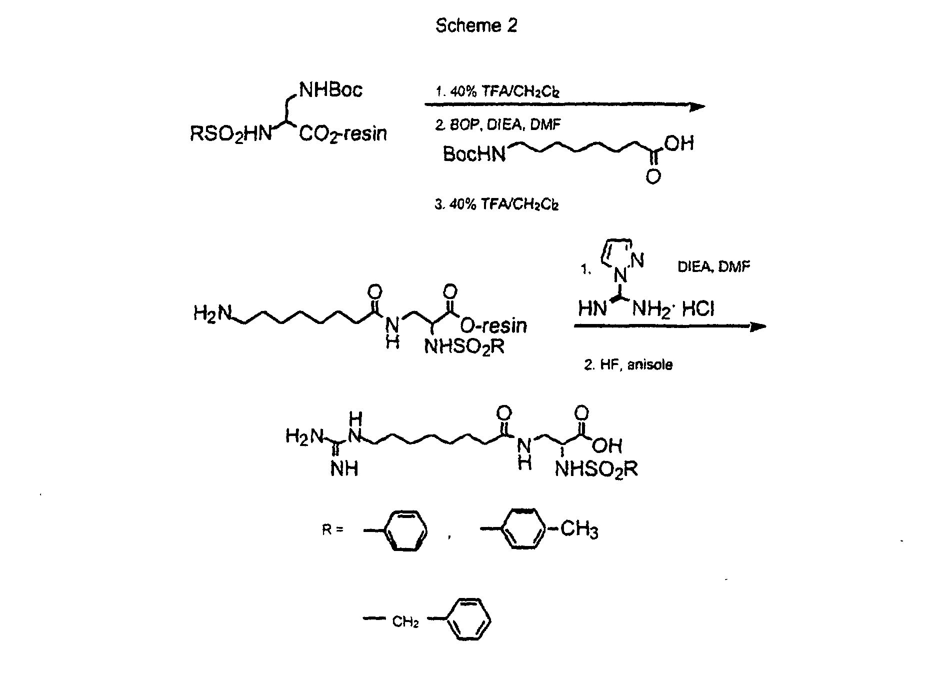

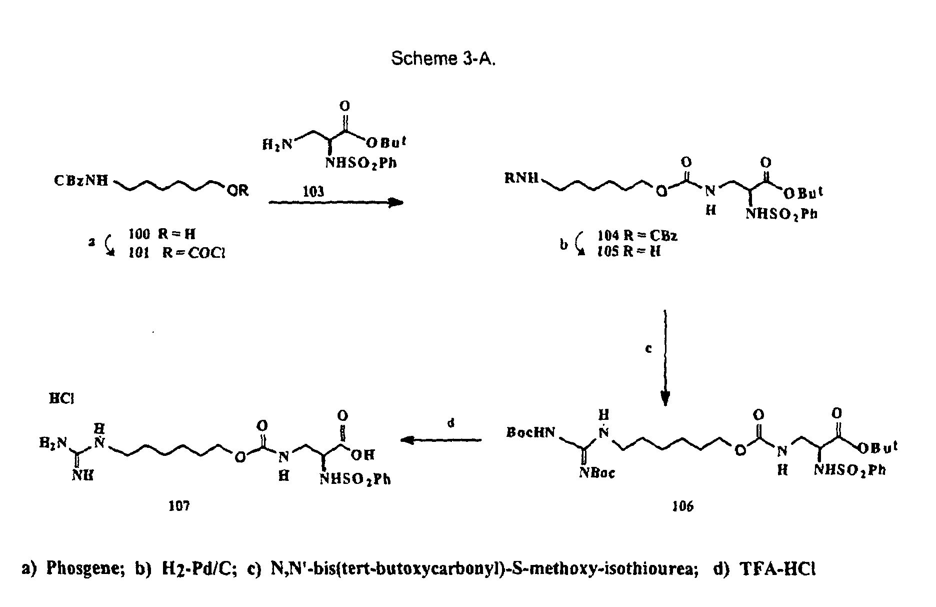

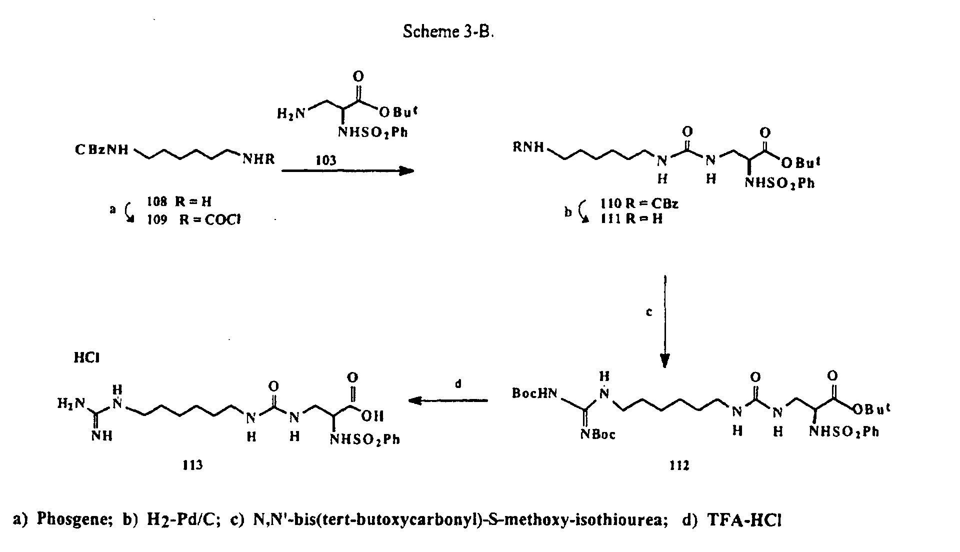

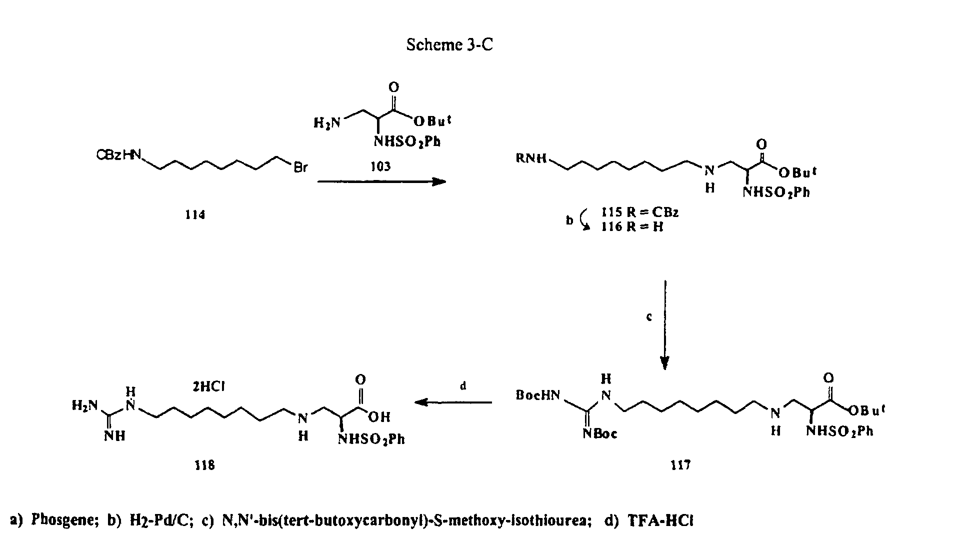

The preparation of the carbamate containing compound **107** typifies the construction of compounds containing this linkage (Scheme 3-A). In the first step, a suitable protected amino alcohol (illustrated with analog **100**) is allowed to react with excess phosgene to provide the intermediate chloroformate (**101**). This material is condensed with the differentially protected diamino propionate derivative **103**, thus producing the carbamate linked intermediate (**104**). The CBz group on the terminal amine is then removed with palladium and hydrogen (**105**) and the formed amine is reacted with N,N'-bis(tert-butoxycarbonyl)-S-methoxy-isothiourea producing the protected guanidine derivative (**106**). The compound is completely deprotected with neat TFA at room temperature and then subjected to a salt exchange with HCI providing the desired amino acids (**107**).

Compounds containing a urea linkage (**113**) are prepared in an analogous manner starting from differentially protected diamines (Scheme 3-B).

Compounds that contain an amine linkage (**118**) can be prepared in a similar fashion (Scheme 3-C). Protected bromo-amine **114** (prepared from 8-(N-carbobenzoxy)aminooctanol by treatment with CBr₄ and Ph₃P) is allowed to react with amine **103** in the presence of k₂CO₃ which forms adduct **115.** Subjection of protected derivative **115** to the same sequence of reactions outlined for the transformation of **104** into **107** affords the desired amine containing compound **118.**

### Compositions and Formulations

The compounds of this invention may be isolated as the free acid or base or converted to salts of various inorganic and organic acids and bases. Such salts are within the scope of this invention. Non-toxic and physiologically-compatible salts are particularly useful although other less desirable salts may have use in the processes of isolation and purification.

A number of methods are useful for the preparation of the salts described above and are known to those skilled in the art. For example, reaction of the free acid or free base form of a compound of the structures recited above with one or more molar equivalents of the desired acid or base in a solvent or solvent mixture in which the salt is insoluble, or in a solvent like water after which the solvent is removed by evaporation, distillation or freeze drying. Alternatively, the free acid or base form of the product may be passed over an ion exchange resin to form the desired salt or one salt form of the product may be converted to another using the same general process.

Diagnostic applications of the compounds of this invention will typically utilize formulations such as solution or suspension. In the management of thrombotic disorders the compounds of this invention may be utilized in compositions such as tablets, capsules or elixirs for oral administration, suppositories, sterile solutions or suspensions or injectable administration, and the like, or incorporated into shaped articles. Subjects in need of treatment (typically mammalian) using the compounds of this invention can be administered dosages that will provide optimal efficacy. The dose and method of administration will vary from subject to subject and be dependent upon such factors as the type of mammal being treated, its sex, weight, diet, concurrent medication, overall clinical condition, the particular compounds employed, the specific use for which these compounds are employed, and other factors which those skilled in the medical arts will recognize.

Formulations of the compounds of this invention are prepared for storage or administration by mixing the compound having a desired degree of purity with physiologically acceptable carriers, excipients, stabilizers etc., and may be provided in sustained release or timed release formulations. Acceptable carriers or diluents for therapeutic use are well known in the pharmaceutical field, and are described, for example, in *Remington's Pharmaceutical Sciences,* Mack Publishing Co., (A.R. Gennaro edit. 1985). Such materials are nontoxic to the recipients at the dosages and concentrations employed, and include buffers such as phosphate, citrate, acetate and other organic acid salts, antioxidants such as ascorbic acid, low molecular weight (less than about ten residues) peptides such as polyarginine, proteins, such as serum albumin, gelatin, or immunoglobulins, hydrophilic polymers such as polyvinylpyrrolidinone, amino acids such as glycine, glutamic acid, aspartic acid, or arginine, monosaccharides, disaccharides, and other carbohydrates including cellulose or its derivatives, glucose, mannose or dextrins, chelating agents such as EDTA, sugar alcohols such as mannitol or sorbitol, counterions such as sodium and/or nonionic surfactants such as Tween, Pluronics or polyethyleneglycol.

Dosage formulations of the compounds of this invention to be used for therapeutic administration must be sterile. Sterility is readily accomplished by filtration through sterile membranes such as 0.2 micron membranes, or by other conventional methods. Formulations typically will be stored in lyophilized form or as an aqueous solution. The pH of the preparations of this invention typically will be between 3 and 11, more preferably from 5 to 9 and most preferably from 7 to 8. It will be understood that use of certain of the foregoing excipients, carriers, or stabilizers will result in the formation of cyclic polypeptide salts. While the preferred route of administration is by injection, other methods of administration are also anticipated such as intravenously (bolus and/or infusion), subcutaneously, intramuscularly, colonically, rectally, nasally or intraperitoneally, employing a variety of dosage forms such as suppositories, implanted pellets or small cylinders, aerosols, oral dosage formulations and topical formulations such as ointments, drops and dermal patches. The compounds of this invention are desirably incorporated into shaped articles such as implants which may employ inert materials such as biodegradable polymers or synthetic silicones, for example, Silastic, silicone rubber or other polymers commercially available.

The compounds of this invention may also be administered in the form of liposome delivery systems, such as small unilamellar vesicles, large unilamellar vesicles and multilamellar vesicles. Liposomes can be formed from a variety of lipids, such as cholesterol, stearylamine or phosphatidylcholines.

The compounds of this invention may also be delivered by the use of antibodies, antibody fragments, growth factors, hormones, or other targeting moieties, to which the compound molecules are coupled. The compounds of this invention may also be coupled with suitable polymers as targetable drug carriers. Such polymers can include polyvinylpyrrolidinone, pyran copolymer, polyhydroxy-propyl-methacrylamide-phenol, polyhydroxyethyl-aspartamide-phenol, or polyethyleneoxide-polylysine substituted with palmitoyl residues. Furthermore, inhibitors of this invention may be coupled to a class of biodegradable polymers useful in achieving controlled release of a drug, for example polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels. Polymers and semipermeable polymer matrices may be formed into shaped articles, such as valves, stents, tubing, prostheses and the like.

Therapeutic compound liquid formulations generally are placed into a container having a sterile access port, for example, an intravenous solution bag or vial having a stopper pierceable by hypodermic injection needle.

Therapeutically-effective dosages may be determined by either *in vitro* or *in vivo* methods. For each particular compound of the present invention, individual determinations may be made to determine the optimal dosage required. The range of therapeutically-effective dosages will naturally be influenced by the route of administration, the therapeutic objectives, and the condition of the patient. For injection by hypodermic needle, it may be assumed the dosage is delivered into the body's fluids. For other routes of administration, the absorption efficiency must be individually determined for each inhibitor by methods well known in pharmacology. Accordingly, it may be necessary for the therapist to titer the dosage and modify the route of administration as required to obtain the optimal therapeutic effect. The determination of effective dosage levels, that is, the dosage levels necessary to achieve the desired result, will be within the ambit of one skilled in the art. Typically, applications of compound are commenced at lower dosage levels, with dosage levels being increased until the desired effect is achieved.

A typical dosage might range from about 0.001 mg/kg to about 1000 mg/kg, preferably from about 0.01 mg/kg to about 100 mg/kg, and more preferably from about 0.10 mg/kg to about 20 mg/kg. Advantageously, the compounds of this invention may be administered several times daily, and other dosage regimens may also be useful.

Typically, about 0.5 to 500 mg of a compound or mixture of compounds of this invention, as the free acid or base form or as a pharmaceutically-acceptable salt, is compounded with a physiologically acceptable vehicle, carrier, excipient, binder, preservative, stabilizer, dye, flavor etc., as called for by accepted pharmaceutical practice. The amount of active ingredient in these compositions is such that a suitable dosage in the range indicated is obtained.

Typical adjuvants which may be incorporated into tablets, capsules and the like are a binder such as acacia, corn starch or gelatin, and excipient such as microcrystalline cellulose, a disintegrating agent like corn starch or alginic acid, a lubricant such as magnesium stearate, a sweetening agent such as sucrose or lactose, or a flavoring agent. When a dosage form is a capsule, in addition to the above materials it may also contain a liquid carrier such as water, saline, or a fatty oil. Other materials of various types may be used as coatings or as modifiers of the physical form of the dosage unit. Sterile compositions for injection can be formulated according to conventional pharmaceutical practice. For example, dissolution or suspension of the active compound in a vehicle such as an oil or a synthetic fatty vehicle like ethyl oleate, or into a liposome may be desired. Buffers, preservatives, antioxidants and the like can be incorporated according to accepted pharmaceutical practice.

In practicing the methods of this invention, the compounds of this invention may be used alone, or in combination with other therapeutic or diagnostic agents. In certain preferred embodiments, the compounds of this invention may be coadministered along with other compounds typically prescribed for these conditions according to generally accepted medical practice, such as anticoagulant agents, thrombolytic agents, or other antithrombotics, including platelet aggregation inhibitors, tissue plasminogen activators, urokinase, prourokinase, streptokinase, heparin, aspirin, or warfarin. The compounds of this invention can be utilized *in vivo,* ordinarily in mammals such as primates, (e.g. humans), sheep, horses, cattle, pigs, dogs, cats, rats and mice, or *in vitro.*

With respect to the coronary arterial vasculature, abnormal thrombus formation characterizes the rupture of an established atherosclerotic plaque which is the major cause of acute myocardial infarction and unstable angina, as well as also characterizing the occlusive coronary thrombus formation resulting from either thrombolytic therapy or percutaneous transluminal coronary angioplasty (PTCA).

The compounds of the present invention preferably have an IC₅₀ of less than about 2.0 µM, more preferably less than about 1.0 µM, and most preferably less than about 200 nM, as measured by one or more of the assays described herein.. These compounds, selected and used as disclosed herein, are believed to be useful for preventing or treating a condition characterized by undesired thrombosis, such as, by way of illustration and not limitation, (a) the treatment or prevention of any thrombotically mediated acute coronary syndrome including myocardial infarction, unstable angina, refractory angina, occlusive coronary thrombus occurring post-thrombolytic therapy or post-coronary angioplasty, (b) the treatment or prevention of any thrombotically mediated cerebrovascular syndrome including embolic stroke, thrombotic stroke or transient ischemic attacks, (c) the treatment or prevention of any thrombotic syndrome occurring in the venous system including deep venous thrombosis or pulmonary embolus occurring either spontaneously or in the setting of malignancy, surgery or trauma, thrombotic thrombocytopenic purpura, thromboangiitis obtiterans, or thrombotic disease associated with heparin induced thrombocytopenia, (e) the treatment or prevention of thrombotic complications associated with extracorporeal circulation (e.g. renal dialysis, cardiopulmonary bypass or other oxygenation procedure, plasmapheresis), (f) coagulopathy and disseminated intravascular coagulation (g) the treatment or prevention of thrombotic complications associated with instrumentation (e.g. cardiac or other intravascular catheterization, intra-aortic balloon pump, coronary stent or cardiac valve), (h) those involved with the fitting of prosthetic devices, (i) vascularization of solid tumors and (j) retinopathy.

Without further description, it is believed that one of ordinary skill in the art can, using the preceding description and the following illustrative examples, make and utilize the compounds of the present invention and practice the claimed methods. The following working examples therefore, specifically point out preferred embodiments of the present invention, and are not to be construed as limiting in any way the remainder of the disclosure.

### EXAMPLES

### Example 1

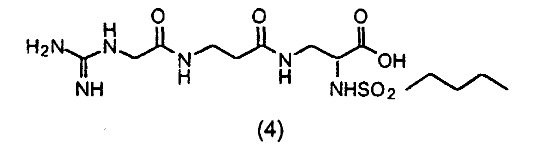

### Part A: Synthesis of 2-(n-Butylsulfonylamido)-3-(N-Boc)-aminopropionic acid hydroxymethyl resin (1)

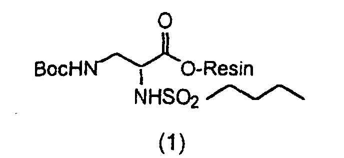

Hydroxymethyl resin (2.7 g, 2.1 mmol, 0.8 mmol/ g) was suspended in DMF (20 ml 2-(n-Butylsulfonylamido)-3-(N-Boc)-aminopropionic acid ( prepared according to Claremor D. A., *et al*., PCT/US94/01881) (1.0 g, 3.0 mmol), BOP (2.8 g, 6.3 mmol), DIEA (2.0 ml, 11.1 mmol) and catalytic amount of DMAP were added. The mixture was rotated on a nutator at r.t. overnight. The resulting resin (1) was collected in a fritted glass Buchner funnel, washed with MeOH, CH₂Cl₂ and dried in vacuo.

### Part B:

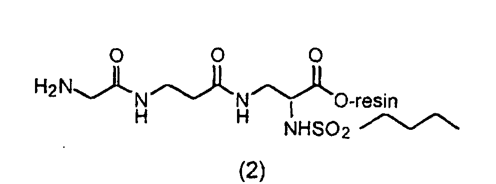

Compound (2) was synthesized by standard automated solid-phase synthesis protocols using an Applied Biosystems 431 A Peptide Synthesizer. The resin (1) was deprotected with 50% TFA/CH₂Cl₂ followed by coupling with N-Boc-β-alanine. The Boc group was removed again with 50% TFA/CH₂Cl₂ followed by coupling with N-Boc-glycine. The Boc group was removed with TFA and the resin dried.

### Part C:

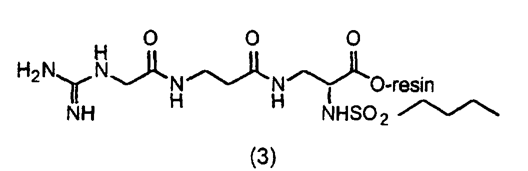

The resin (2) (0.5 mmol of peptide theoretical) was neutralized with 10% DIEA/CH₂Cl₂ and swelled and saturated with DMF (15 ml). 1H-Pyrazole-1-carboxamidine hydrochloride (2 g, 13.8 mmol) and DIEA (2.9 ml, 15.7 mmol) were added to the resin mixture. The reaction was allowed to proceed at 37° for 2 hr. after which the Kaiser ninhydrin test of a resin sample was negative. The resulting resin was washed with CH₂Cl₂ and CH₃OH and dried in *vacuo.*

### Part D: HF Cleavage of the resin (3).

The resin (3) (1 g) was suspended in HF (10 ml/g resin) containing 10% by volume anisole and 2% methylethylsulfide (MES) employing a type 1B HF cleavage apparatus. The liquid HF was condensed into the cleavage vessel with the aid of liquid nitrogen cooling and was then maintained at -10° C for 30 min and at 0°C for an additional 30 min. The HF was removed in vacuo and the resin transferred to a fritted glass funnel. The resin was washed with ether followed by extraction of the crude product from the resin with 2N aqueous acetic acid. Lyophilization of the extracts furnished a white powder. The product (4) was purified by preparative HPLC. MS (ES) 395 (M+H⁺)

### Example 2

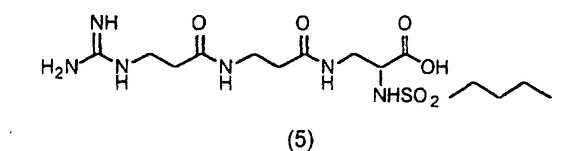

Compound (5) was synthesized by the method of Example 1 using N-Boc-β-alanine in place of N-Boc-glycine in Part B. MS (ES) 409 (M+H⁺)

### Example 3

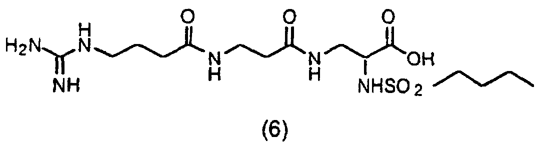

Compound (6) was synthesized by the method of Example 1 using N-Boc-4-aminobutyric acid in place of N-Boc-glycine in Part B. MS (ES) 423 (M+H⁺)

### Example 4

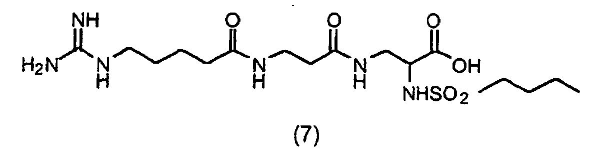

Compound (7) was synthesized by the method of Example 1 using N-Boc-5-aminovaleric acid in place of N-Boc-glycine in Part B. MS (ES) 437 (M+H⁺)

### Example 5

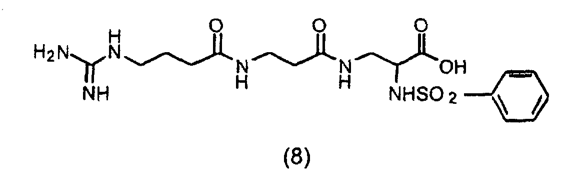

Compound (8) was synthesized by the method of Example 1 using 2-(benzenesulfonylamido)-3-(N-Boc)-aminopropionic acid in place of 2-(n-butylsulfonylamido)-3-(N-Boc)-aminopropionic acid. MS (ES) 443 (M+H⁺). 2-(benzenesulfonylamido)-3-(N-Boc)-aminopropionic acid is prepared as described by Claremon, D. A., *et al.,* PCT/US94101881, as follows for the preparation of sulfonamide intermediate compounds:

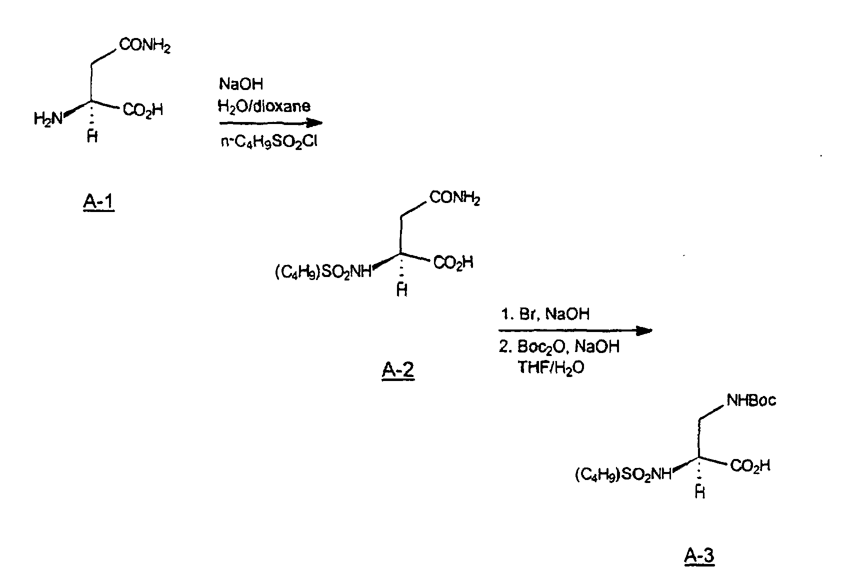

### L-Asparagine-α-butanesulfonamide (also N-(n-Butyl-sulfonyl)-L-asparagine) A-2

A solution containing L-asparagine (6.45 g, 48.9 mmol) and NaOH (2.0 g, 50.0 mmol) in 100 ml of 50% aqueous dioxane was cooled to 0°C in an ice bath. To this rapidly stirred mixture, a solution of NaOH (2.2 g, 55.0 mmol) in 50 mL of water and neat butane sulfonyl chloride (7.0 ml, 53.9 mmol) were added alternately over a period of 30 min. The reaction solution was concentrated to a volume of 50 ml at reduced pressure and aqueous residue was cooled, acidified with concentrated HCI, and extracted into ethyl acetate (3 x 100 ml). The organic extracts were dried over Na2SO4 and concentrated to a volume of approximately 50 ml, anhydrous ether (50 ml) was added and the resulting white precipitate was isolated by vacuum filtration yielding A-2, mp. 154-155°C.

### L-β Boc-α-butane sulfonamido-β amino alanine (also 2(S)-(n-Butyl-sulfonylamino)-3-(N-Boc-aminopropionic acid) A-3

A solution containing NaOH (6.04 g, 151 mmol) in 50 ml H20 was cooled to 0°C and bromine (1.40 ml, 26.9 mmol) was added. The resulting solution was stirred at 0°C for 5 min. Next, a cooled solution of A-2 (5.23 g, 20.7 mmol) and NaOH (1.66 g, 41.4 mmol) in 15 ml of H₂O was added at once and mixture stirred at 0°C for 5 min then heated to 80°C for 15 min. The solution was then cooled to 25°C and acidified with 12N HCl (11 ml) and stirred until gas evolution ceased. The solution was then made basic by the addition of 2N NaOH and 20 ml of THF was added along with di-t-butyl dicarbonate (9.0 g, 41.4 mmol). After stirring overnight at 25°C the THF was removed at reduced pressure and the basic aqueous phase extracted with ethyl acetate (2 x 50 ml). The aqueous phase was then made acidic with 10% KHSO₄ and extracted with ethyl acetate (3 x 100 ml). The pooled acidic extracts were dried over Na₂SO₄ filtered and evaporated giving A-3 as a white solid, mp 111-112°C.

### Example 6

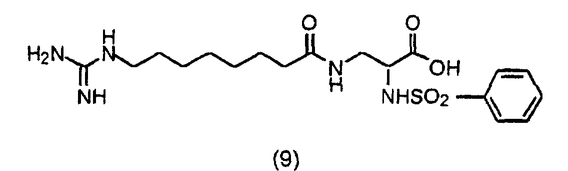

### PART A:

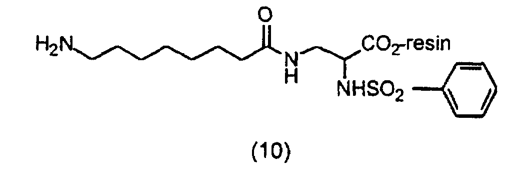

To the 2-(benzenesulfonylamido)-3-(N-Boc)-aminopropionic acid hydroxymethyl resin from Example 5 (0.75 g, 0.375 mmol) in a fritted reaction vessel, was added 40% TFA/CH₂Cl₂. The mixture was stirred at r.t. for 30 min. The resulting 2-(benzenesulfonylamido)-3-aminopropionic acid hydroxymethyl resin was collected through filtration, washed with CH₂Cl₂ and MeOH repeatedly and neutralized with 10% DIEA in CH₂Cl₂. Then, a solution of 8-(N-Boc) aminooctanoic acid (0.195 g, 0.75 mmol), BOP (0.5 g, 1.1 mmol) and DIEA (0.35 ml, 1.9 mmol) in 2 ml of DMF was added to the neutralized resin. The reaction proceeded on a nutator at r.t. for 8 hr., after which the Kaiser ninhydrin test of a resin sample was negative. The mixture was filtered and the resin collected. The resin was washed with CH₂Cl₂, MeOH, dried in vacuo and the Boc group cleaved with 40% TFA/CH₂Cl₂. The resulting free amino-containing resin was washed and dried in vacuo to yield titled compound (10).

### Part B:

Compound (9) was synthesized by the method of Part C and D of Example 1 using (10) in place of (2) in Part B. MS (ES) 428 (M+H⁺)

### Example 7

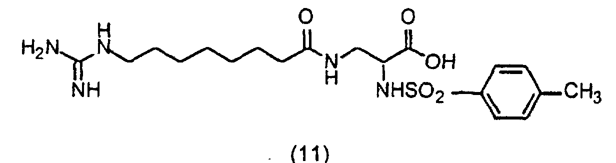

Compound (11) was synthesized by the method of Example 6 using *p*-toluenesulfonyl chloride in place of benzenesulfonyl chloride. MS (ES) 442 (M+H⁺)

### Example 8

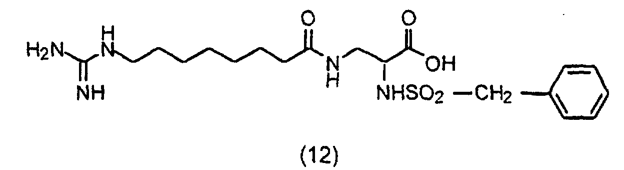

Compound (12) was synthesized by the method of Example 6 using α-toluenesulfonyl chloride in place of benzenesulfonyl chloride. MS (ES) 442 (M+H⁺)

### Example 9

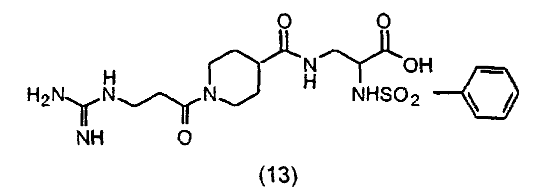

Compound (13) was synthesized by the method of Part B, C, and D of Example 1 using N-Boc-β-alanine, N-Boc-isonipecotic acid and 2-(benzenesulfonylamido)-3-(N-Boc)-aminopropionic acid hydroxymethyl resin. MS (ES) 469 (M+H⁺)

### Example 10

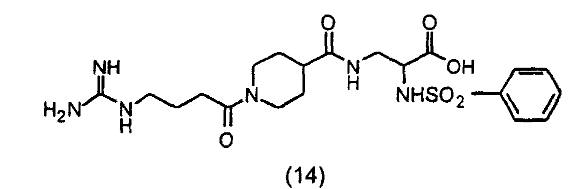

Compound (14) was synthesized by the method of Example 9 using N-Boc-4-aminobutyric acid in place of N-Boc-β-alanine. MS (ES) 483 (M+H⁺)

### Example 11

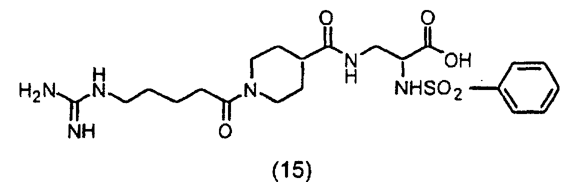

Compound (15) was synthesized by the method of Example 9 using N-Boc-5-aminovaleric acid in place of N-Boc-β-alanine. MS (ES) 497 (M+H⁺)

### Example 12

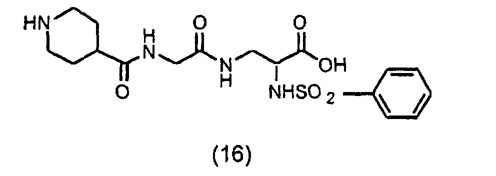

Compound (16) was synthesized by the method of Example 9 (omitting the step of guanylating the nitrogen) using N-Boc-isonipecotic acid in place of N-Boc-β-alanine at the N-terminus and N-Boc-glycine in place of N-Boc-isonipecotic acid as the central spacer. MS (ES) 413 (M+H⁺)

### Example 13

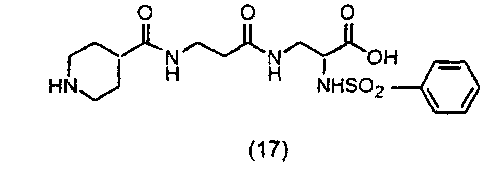

Compound (17) was synthesized by the method of Example 12 using N-Boc-β-alanine in place of N-Boc-glycine. MS (ES) 427 (M+H⁺)

### Example 14

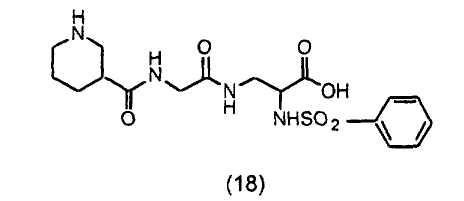

Compound (18) was synthesized by the method of Example 12 using N-Bocnipecotic acid in place of N-Boc-isonipecotic acid. MS (ES) 413 (M+H⁺)

### Example 15

### Part A:

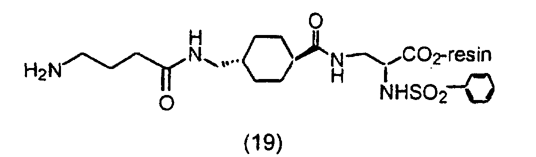

Compound (19) was synthesized by the method of Part A of Example 6 using trans-(Boc-4-aminomethyl)-cyclohexanecarboxylic acid and N-Boc-4-aminobutyric acid in place of 8-(N-Boc)aminooctanoic acid and using HBTU in place of BOP.

### Part B:

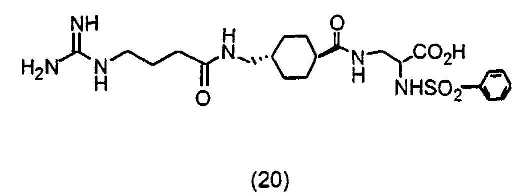

Compound (20) was synthesized from compound (19) by the method of part C and D of Example 1. MS (ES) 511 (M+H)⁺

### Example 16

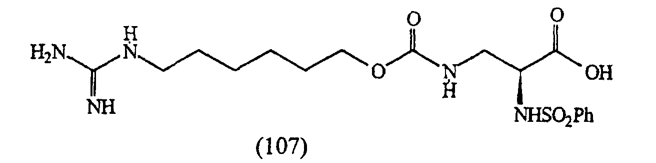

### Preparation of 107 (Scheme 3-A)

### Step 1.

A mixture of CBz protected amino-hexanol **100** (0.10 g, 0.39 mmol) was dissolved in toluene (5 mL) and treated with a 2M solution of phosgene in toluene (2 mL). This solution was allowed to stir for 2 h at room temperature and was then concentrated to dryness. The residue was then dissolved in CH₂Cl₂ (5 mL) and added to a solution of **103** (0.0.12 g, 0.39 mmol), pyridine (1 mL) and CH₂Cl₂ (5 mL). The resulting solution was allowed to stir for 2 h. The solution was then diluted with EtOAc (100 mL) and washed with saturated NaHCO₃ and water. The organic material was then dried (MgSO₄) an concentrated. The crude residue was purified by chromatography (silica gel, Hexanes-EtOAc 1:1) giving 0.213 g of **104**.

### Step 2.

A mixture of compound **104** (0.213 g), 10% palladium on carbon (0.2g), and ethanol (10 mL) was stirred under an atmosphere of hydrogen for 2h and then filtered and concentrated providing 0.14 g of the desired amine **105.**

### Step 3.

A mixture of the amine **105** (0.14 g, 0.32 mmol), N,N'-bis(tert-butoxycarbonyl)-S-methoxy-isothiourea (0.147 g, 0.49 mmol), and CH₂Cl₂ (1 mL) was maintained at room temperature for 48 h and then was concentrated. This material was then purified by chromatography (silica gel, hexanes-EtOAc - 1:1) providing **106.**

### Step 4.

A mixture of the **106** (0.1 g) was dissolved in anhydrous TFA (5 mL) and maintained at room temperature for 1h. This material was then concentrated to dryness and the resulting material was taken up in 2N HCl (5 mL) and lyophilized providing **107** as a white hygroscopic solid. MS (m/e) 430 (MH+).

### Example 17

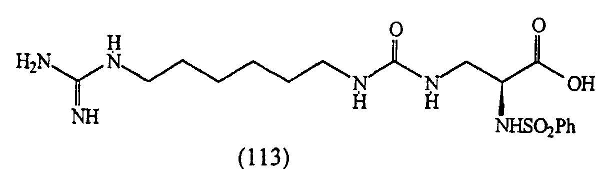

### Preparation of 113 (Scheme 3-B)

Mono CBz protected hexane diamine **(108)** was converted to the urea derivative **110** following the same procedure employed for the preparation of **104** (Example 16 step 1). Compound **110** was further transformed by first removing the CBz protecting group using the procedure outlined in Example 16 step 2 which afforded amine **111**. This material was converted to the protected guanidine **112** using the procedure outlined in Example 16 step 3. Finally the desired material (**113**) was obtained using the procedure outlined in Example 16 step 4. MS (m/e) 428 (MH+).

### Example 18

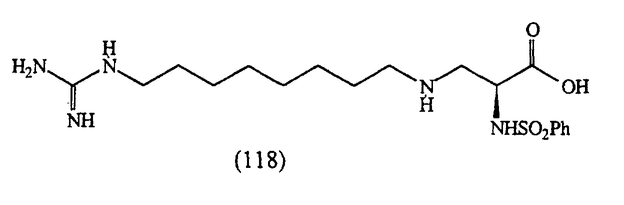

### Preparation of 118 (Scheme 3-C)

A mixture of the bromide **114** (0.11g, 0.33 mmol), amine **103** (0.10 g, 0.33 mmol), and K₂CO₃ (0.05 g, 0.33 mmol) in CH₃CN (1 mL) was stirred at 50°C overnight. The mixture was then diluted with EtOAc (50 ml) and washed with H₂O. The organic material was dried and concentrated. Chromatography (silica gel - EtOAc) gave 0.05 g of **115** as a clear oil. Compound **115** was further transformed by first removing the CBz protecting group using the procedure outlined in Example 16 step 2 which afforded amine **116**. This material was converted to the protected guanidine **117** using the procedure outlined in Example 16, step 3. Finally the desired material (**118**) was obtained using the procedure outlined in Example 16, step 4. MS (m/e) 414 (MH+).

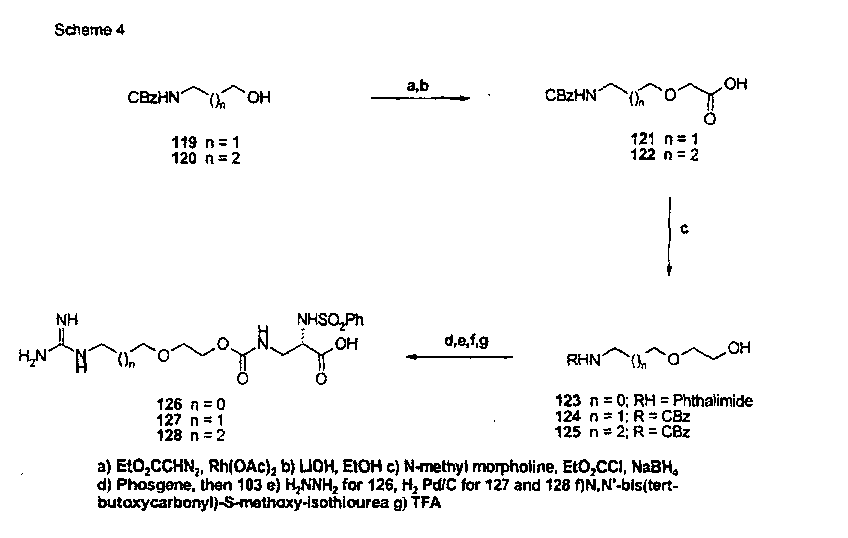

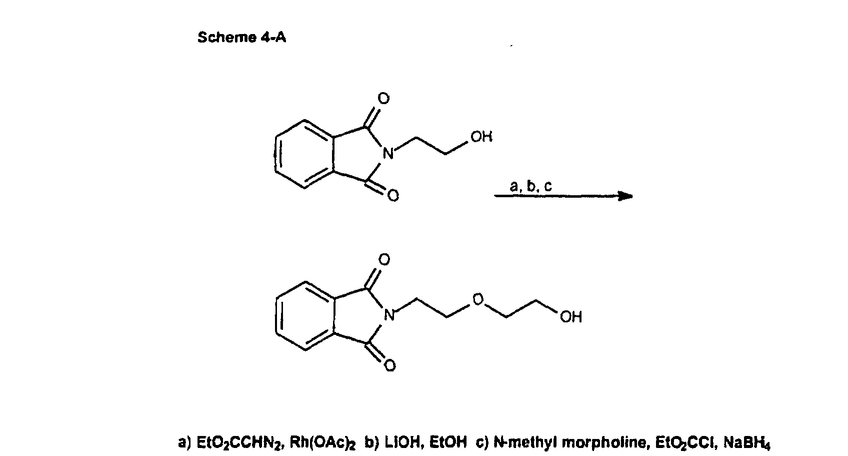

Scheme 4-A depicts a metal catalyzed carbene insertion reaction suitable for preparing the starting material **123.**

### Example 19

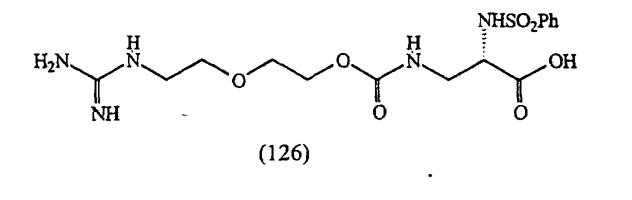

### Preparation of 126 (Scheme 4)

2-(2-Phthaloylethoxy)ethanol **123** (200mg, 0.85mmol) is prepared by Scheme 4-A and is thereafter treated with phosgene (2.2mL, 1.93M in toluene, 4.3mmol). After 1h, the mixture is concentrated and the residue dissolved in pyridine (2mL). This mixture is then added dropwise to a solution of amine **103** (255mg, 0.85mmol) in pyridine (2mL). After 1h, the mixture is poured into H₂O and ethyl acetate (EtOAc). The layers are separated, the aqueous layer washed with EtOAc (2x), and the combined extracts are dried (MgSO₄) and concentrated. The residue is purified by chromatography (SiO₂, 1:1 hexane:EtOAc) affording 180mg (32%) of the desired carbamate. Deprotection of the phthalimide is accomplished by treatment of the carbamate product with hydrazine hydrate (0.80mL) in ethanol (2mL). After 1h, the mixture is concentrated, partitioned between NaHCO₃(aq) and EtOAc, and the layers are separated. The organic extract is washed with NaHCO₃(aq)(2x), dried (MgSO₄), and concentrated yielding 66mg (48%) of the corresponding amine. The protected guanidine is prepared according to a similar procedure described for the preparation of **107** (Scheme 1). Deprotection with trifluoroacetic acid (TFA) yields the TFA salt of the desired product **126.** MS (m/e) 418 (MH+).

### Example 20

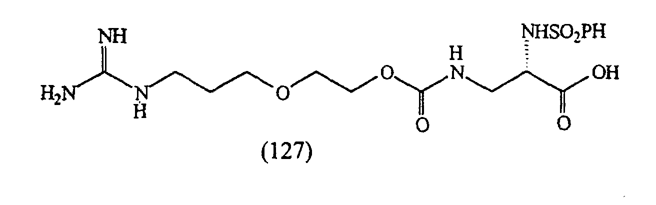

### Preparation of 127 (Scheme 4)

### Step 1.

A solution of CBz protected 3-aminopropanol **119** (500mg, 0.24mmol) and rhodium(II) acetate dimer (10mg) in CH₂Cl₂ (40mL) was treated dropwise with a CH₂Cl₂ solution of ethyl diazoacetate (0.28mL, 2.63mmol). After 1h, the mixture was concentrated and the residue was purified by flash chromatography (SiO₂, 1:1 hexane:EtOAc) affording 560mg (79%) of the ethyl ester. The ester (300mg) was dissolved in EtOH (10mL) and treated with NaOH (203mg, 0.51mmol). After 1h, the mixture was concentrated and the residue dissolved in H₂O. The pH was adjusted to -2-3 using 1N HCI and then EtOAc was added and the layers were separated. The aqueous layer was washed with EtOAc (2x), the combined extracts were then dried (MgSO₄) and concentrated yielding 227mg (83%) of acid **121.**

### Step 2.

A solution of acid **121** (227mg, 0.085mmol) and N-methyl morpholine (0.94mL, 0.085mmol) in THF (4.5mL) at -10C was treated with ethyl chloroformate (0.82mL, 0.085mmol). After 0.25h, the mixture was treated with sodium borohydride (96mg, 0.26mmol) followed by dropwise addition of methanol (9mL). The mixture was allowed to warm, treated with 10% acetic acid (aq), and concentrated. The residue was partitioned between 1N NaOH and EtOAc, the layers were seperated, the aqueous was washed with EtOAc (2x) and the combined extracts were dried (MgSO4) and concentrated. The residue was purified by chromatography (SiO2, 65:35 EtOAc:hexane) yielding 75mg (35%) of alcohol **124.**

### Step 3.

The carbamate derivative **127** was prepared following the same procedures described for the preparation of **107** (Scheme 1). Alcohol **124** (75mg) was treated with phosgene followed by amine **103** (89mg) yielding the carbamate (63mg). Deprotection of the CBz by hydrogenolysis and subsequent treatment of the resulting amine (50mg) with N,N'-bis(*tert*-butoxycarbonyl)-S-methoxy-isothiourea (35mg) yielded the protected guanidine (30mg). Deprotection with trifluoroacetic acid yielded the TFA salt of the desired product **127**. MS (m/e) 432 (MH+).

### Example 21

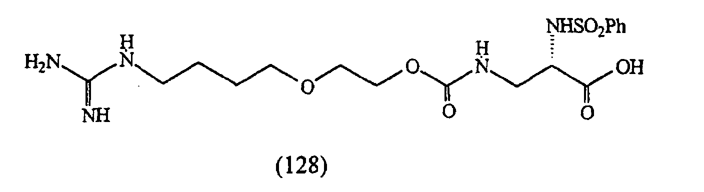

### Preparation of 128 (Scheme 4)

The product **128** was prepared following the same general procedures described for the preparation of **127.** CBz protected 4-aminobutanol (1.0g) was treated with rhodium(II) acetate dimer (10mg) and ethyl diazoacetate (0.52mL) yielding the homologated ester (440mg). Saponification of the ester (728mg) with NaOH (470mg) and reduction of the resulting acid **122** (590mg) with ethyl chloroformate (0.20mL), N-methyl morpholine (0.23mL) and sodium borohydride (238mg) yielded alcohol **125** (134mg). Treatment of **125** with phosgene followed by amine **103** (151mg) yielded the desired carbamate (127mg). Deprotection of the CBz by hydrogenolysis and subsequent treatment of the resulting amine (42mg) with N,N'-bis(*tert*-butoxycarbonyl)-S-methoxy-isothiourea (30mg) yielded the protected guanidine (23mg). Deprotection with trifluoroacetic acid yielded the TFA salt of the desired product **128.** MS (mle) 446 (MH+).

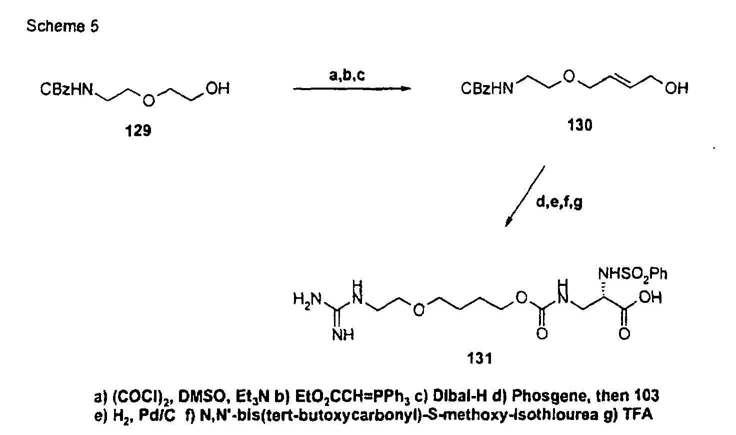

### Example 22

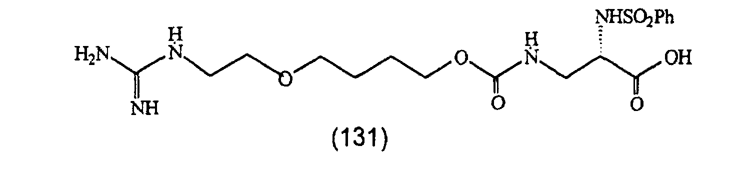

### Preparation of 131 (Scheme 5)

### Step 1.

A solution of oxalyl chloride (0.99mL, 11.3mmol) in CH₂Cl₂ (40mL) at -78°C was treated with dimethyl sulfoxide (0.86mL, 12.2mmol). After 0.5h, a solution of alcohol **129** (1.94g, 8.12mmol) in CH₂Cl₂ (7mL) was added dropwise. After 0.5h, triethylamine (2.05g, 20.3mmol) was added and the cooling bath was removed. After 1h, the mixture was poured into EtOAc and NH₄Cl(aq). The layers were separated, the aqueous layer was washed with EtOAc and the combined extracts were dried (MgSO₄). Concentration yielded the corresponding aldehyde. A solution of this crude aldehyde in THF (20mL) was then treated with (carbethoxymethylene)triphenylphosphorane (2.96g, 8.50mmol). After 62h, the mixture was concentrated and the residue purified by chromatography (SiO₂, 4:1 to 3:2 hexane:EtOAc) yielding the acrylate as a mixture of isomers. A sample of the *tran*s isomer (490mg, 1.60mmol) in THF (5mL) at -78°C was treated with diisobutylaluminum hydride (DibaI-H) (5mL, 1.0M in toluene, 5.0mmol). After 1,5h, the mixture was treated with EtOAc (5mL) followed by a saturated aqueous solution of NH₄Cl (0.4mL). After 1h, the mixture was treated with SiO₂ gel (∼4mL), diluted with EtOAc, and filtered through MgSO₄ and Celite. Concentration yielded 237mg (56%) of the alcohol **130**.

### Step 2.

The carbamate derivative **131** was prepared following the same procedures described for the preparation of **107** (Example 16, step 1). Alcohol **130** (237mg) was treated with phosgene followed by amine **103** (263mg) yielding the carbamate (368mg). Deprotection of the CBz by hydrogenolysis and subsequent treatment of the resulting amine (116mg) with N,N'-bis(*tert*-butoxycarbonyl)-S-methoxy-isothiourea (147mg) yielded the protected guanidine (104mg, 59%). Deprotection with trifluoroacetic acid yielded the TFA salt which upon treatment with aqueous HCI and lyophilization yielded the HCI salt of the desired product **131.** MS (m/e) 446 (M+).

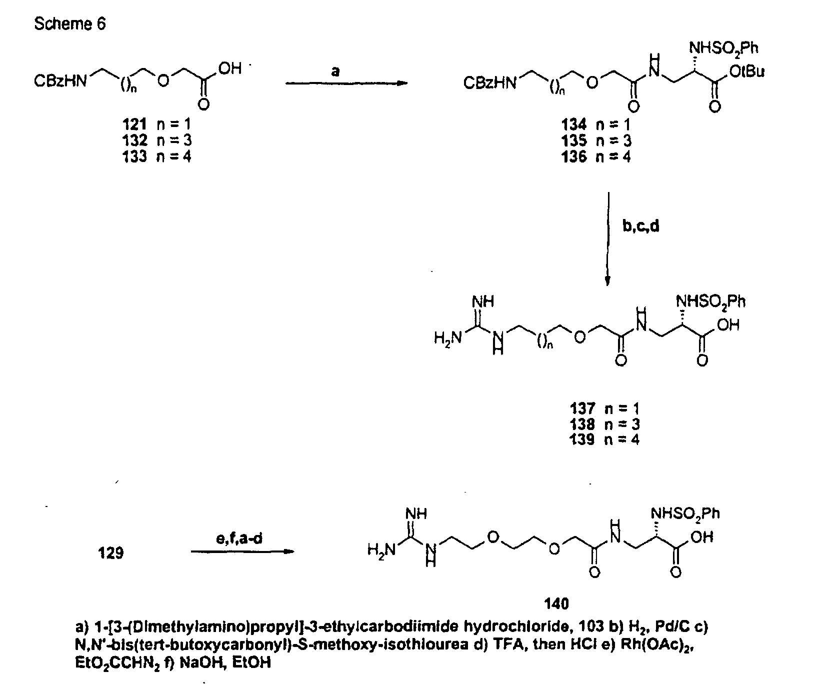

### Example 23

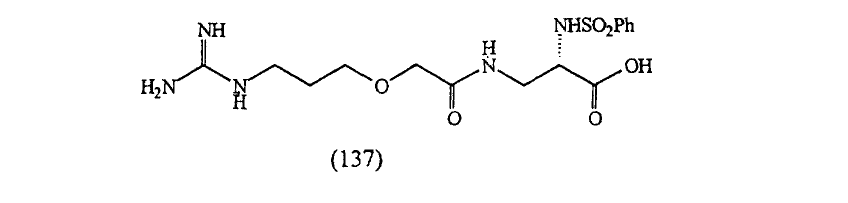

### Preparation of 137 (Scheme 6)

### Step 1.

A solution of acid **121** (Scheme 4, n=1)(418mg, 1.56mmol) and amine **103** (470mg, 1.56mmol) in CH₂Cl₂ (8mL) was treated with 1-[3-(dimethylamino)propyl]-3-ethylcarbodiimde hydrochloride (EDCI) (300mg, 1.56mmol) and triethylamine (0.55mL, 3.9mmol). After 2h, the mixture was concentrated and the residue purified by chromatography (SiO₂, 4:1 EtOAc:hexane) affording 60mg (7%) of the amide **134.**

### Step 2.

The amide derivative **137** was then prepared following the same procedures described for **107** (Scheme 3-A, steps 2-4). Hydrogenolysis of **134** (152mg) yielded the corresponding amine (130mg). Treatment of the amine (111mg) with N,N'-bis(*tert*-butoxycarbonyl)-S-methoxy-isothiourea (85mg) yielded the protected guanidine (100mg, 57%). Deprotection with trifluoroacetic acid yielded the TFA salt which upon treatment with aqueous HCl and lyophilization yielded the HCl salt of the desired product **137.** MS (m/e) 446 (M+).

### Example 24

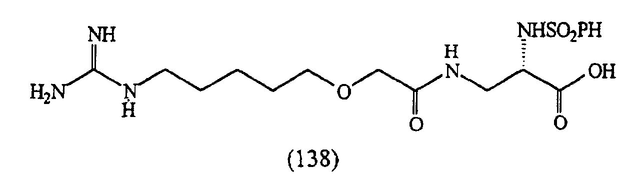

### Preparation of 138 (Scheme 6)

The amide derivative **138** was prepared following the same general procedures described for the preparation of **137** (Scheme 6). Treatment of acid **132** (128mg, prepared according to the rhodium catalyzed method described for **121,** Scheme 4 starting from CBz protected 5-aminopentanol) with amine **103** (130mg) and EDCI (83mg) in CH₂Cl₂ yielded amide 135 (140mg, 56%). Hydrogenolysis of **135** (310mg) yielded the corresponding amine (226mg). Treatment of the amine (210mg) with N,N'-bis(*tert*-butoxycarbonyl)-S-methoxy-isothiourea (155mg) yielded the protected guanidine (160mg, 49%). Deprotection with trifluoroacetic acid yielded the TFA salt which upon treatment with aqueous HCl and lyophilization yielded the HCl salt of the desired product **138.** MS (mle) 430 (M⁺).

### Example 25

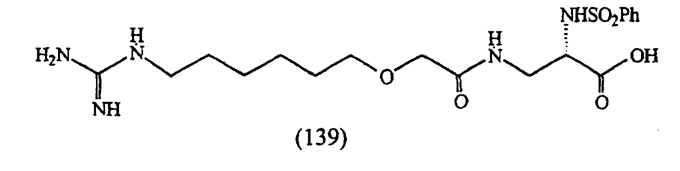

### Preparation of 139 (Scheme 6)

The amide derivative **139** was prepared following the same general procedures described for the preparation of **137** (Scheme 6). Treatment of acid **133** (200mg, prepared according to the rhodium catalyzed method described for **121,** Scheme 4 starting from CBz protected 6-aminohexanol) with amine **103** (195mg), EDCI (124mg), and triethylamine (0.23mL) in CH₂Cl₂ yielded amide **136** (169mg, 44%). Hydrogenolysis of **136** (294mg) yielded the corresponding amine (141mg). Treatment of the amine (120mg) with N,N'-bis(*tert*-butoxycarbonyl)-S-methoxy-isothiourea (84mg) yielded the protected guanidine (55mg, 30%). Deprotection with trifluoroacetic acid yielded the TFA salt which upon treatment with aqueous HCl and lyophilization yielded the HCl salt of the desired product **139**. MS (m/e) 444 (M⁺).

### Example 26

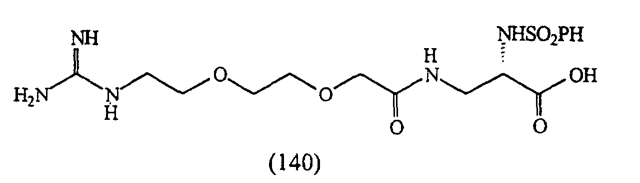

### Preparation of 140 (Scheme 6)

The amide derivative **140** was prepared following the same general procedures described for the preparation of acid **121** (Scheme 4) and amide **137** (Scheme 6). Alcohol **129** (1.45g, 6.07mmol) was treated with ethyl diazoacetate (0.70mL, 6.68mmol) and rhodium(II) acetate dimer (75mg) yielding the corresponding ester (993mg, 50%). Treatment of the ester (525mg) with sodium hydroxide (324mg) in EtOH afforded the acid which was then treated with amine **103** (450mg) and EDCI (311mg) in CH₂Cl₂ (5mL) yielding the amide (865mg, 96%). Hydrogenolysis of the amide (865mg) yielded the corresponding primary amine (583mg). Treatment of the amine (355mg) with N,N'-bis(*tert*-butoxycarbonyl)-S-methoxy-isothiourea (441mg) yielded the protected guanidine (68mg, 11%). Deprotection with trifluoroacetic acid yielded the TFA salt which upon treatment with aqueous HCI and lyophilization yielded the HCl salt of the desired product **140.** MS (m/e) 432 (M+).

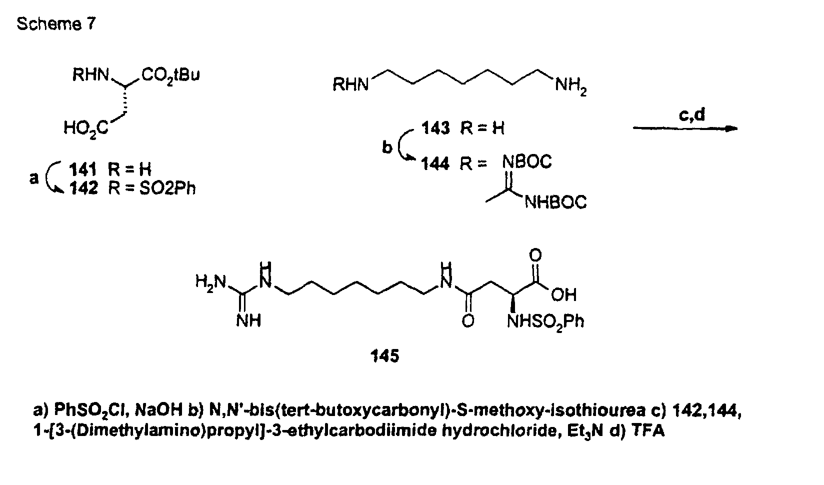

### Example 27

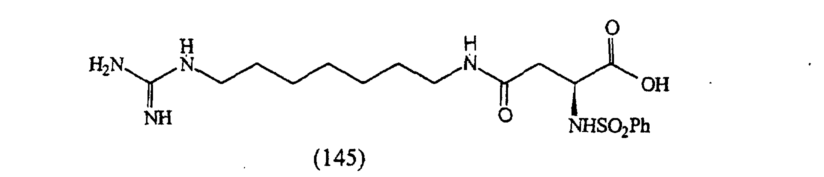

### Preparation of 145 (Scheme 7)

### Step 1.

A solution of **141** (5.0g, 26mmol) in 1:1 H₂O:dioxane (32mL) at 0°C was treated with sodium hydroxide (2.14g, 53mmol) followed by benzenesulfonyl chloride (3.8mL, 29mmol). Upon consumption of **141,** the mixture was concentrated and the pH of the mixture was adjusted to ∼4 by addition of 1N HCI. The aqueous layer was washed with EtOAc (3x) and the combined extracts were dried (MgSO₄) and concentrated yielding the crude sulfonamide **142** which was used without further purification.

### Step 2.

A solution of 1,7-diaminoheptane **143** (3.0g, 23mmol) in DMF (10mL) was treated with N, N'-bis(*tert*-butoxycarbonyl)-S-methoxy-isothiourea (3.3g, 12mmol). The mixture was diluted with H₂O and EtOAc and the layers separated. The aqueous layer was washed with EtOAc (3x) and the combined extracts were washed with 10% citric acid (aq). The aqueous layer was then treated with potassium carbonate until a pH of ∼10 was achieved and then washed with EtOAc (3x). The combined extracts were dried (K₂CO₃) and concentrated to yield the crude amine **144** which was used without further purification.

### Step 3.

A solution of the crude acid **142** (72mg) and amine **144** (123mg) in CH₂Cl₂ was treated with EDCI (42mg) and triethylamine (0.064mL). After 16h, the mixture was concentrated and the residue purified by chromatography (SiO₂, 4:1 to 1:1 hexane:EtOAc) yielding the desired amide (39mg). Deprotection of the amide with trifluoroacetic acid yielded the TFA salt of the desired product **145.** MS (m/e) 428 (MH+).

### Example 28

### Assay Methods

The identification of compounds which are active platelet aggregation inhibitors is made possible by the observation that compounds which block the binding of fibrinogen to the GPIIb-IIIa complex *in vitro* are also capable of inhibiting thrombin or. diphosphate (ADP)-induced aggregation of human platelets and the formation of platelet-thrombi *in vivo.* This observation provides the basis for obtaining potent platelet aggregation inhibitors by evaluating the ability of test materials to disrupt fibrinogen-GPllb-IIIa interactions. The ability of compounds to inhibit the adhesive function of other integrins that are closely related to GPIIb-IIIa can also be measured *in vitro* by measuring their ability to inhibit the binding of adhesive ligands to various integrins such as vitronectin receptor (αᵥβ₃) and fibronectin receptor (α₅β₁).

The following assay methods were used to evaluate the compounds of the invention.

### Integrin Binding Assays:

In the following assays, GPIIb-IIIa and vitronectin receptor, αᵥβ₃ were prepared in purified form, by methods described in Fitzgerald, L.A., *et al.,* Anal Biochem (1985) 151: 169-177 and Smith, J.W., J. Biol Chem (1988) 263: 18726-18731 (the disclosure of which is incorporated herein by reference). GPIIb-IIIa or vitronectin receptor, αᵥβ₃, are coated onto microtiter plates. The coated support is then contacted with fibrinogen for the GPIIb-IIIa assay, or is contacted with vitronectin for the vitronectin receptor, αᵥβ₃ and with test materials and incubated for sufficient time to reach maximal ligand binding to immobilized integrins. The adhesive ligands fibrinogen or vitronectin were typically provided at a concentration of 2-50 nM and the test material can, if desired, be added at a series of diultions. Typical incubations were 2-4 hr at 25°C, the time and temperature being interdependent.

### Description of Purified Integrin Binding Assays

Purified platelet GPIIb-IIIa was prepared by Fitzgerald, L.A., *et al*., Anal Biochem 151:169-177 (1985). Vitronectin receptor, αᵥβ₃, was prepared as described by Smith, J.W., J. Biol Chem (1988) 263: 18726-18731. After purification, the receptors were each stored in 0.1% Triton X-100 at 0.1-1.0 mg/ml.

The receptors were coated to the wells of 96-well flat-bottom enzyme-linked immunoassay (ELISA) plates (Linbro EIA-Plus microtiter plate, Flow Laboratories) after diluting 1:200 with a solution of 20 mM Tris-HCl, 150 mM NaCl, 1 mM CaCl₂, pH 7.4 to reduce the Triton X-100 concentration to below its critical micellar concentration and adding an aliquot of 100 µl to each well. The wells were allowed to incubate overnight at 4°C, and then aspirated to dryness. Additional sites were blocked by the addition of bovine serum albumin (BSA) at 35 mg/ml in the above buffer for 2 hr at 30°C to prevent non-specific binding. The wells were then washed once with binding buffer (50 nM Tris-HCI, 100 mM NaCl, 2 mM CaCl₂, 1 mg/ml BSA).

The corresponding ligands (fibrinogen, von Willebrand Factor, or vitronectin) were conjugated to biotin using commercially available reagents and standard protocols. The labeled ligands were added to the receptor-coated wells at a final concentration of 2-10 nM (100 µl/well) and incubated for 3 hr at 25°C in the presence or absence of test samples. After incubation, the wells were aspirated to dryness and bound ligand containing biotin label was quantitated.

The bound protein was detected by the addition of anti-biotin antibody conjugated to alkaline phosphatase followed by addition of substrate (p-nitrophenyl adenosine phosphate), and determination of the optical density of each well at 405 nM. Decreased color development was observed in wells incubated with test samples which inhibited binding of ligand to receptor. From the various concentrations of test samples, the concentration of inhibitor (IC₅₀) which half-maximally inhibits ligand binding was determined.

### The Platelet Aggregation Assay

In addition to the ELISA integrin binding assays described above, the Aggregation-Human/PRP/ADP Assay is useful for evaluating therapeutic compounds.

Platelet rich plasma (PRP) is prepared from healthy human volunteers for use in determining inhibition of platelet aggregation by compounds. Blood was collected via a 21 gauge butterfly cannula, using a two-syringe technique into 1/10 volume of 105 trisodium citrate.

Platelet rich plasma was prepared at room temperature by centrifugation of the citrated whole blood at 100 x g for 15 minutes. The PRP contained approximately 200-400,000 platelets/µl. Platelet poor plasma was prepared by centrifugation of citrated whole blood at 12,000 x g for 2 minutes.

Platelet aggregation was assayed in a Chrono-log whole blood aggregometer (Chrono-log Corporation, Havertown, PA) using the PRP prepared above according to the manufacturers directions. Inhibition of platelet aggregation was studied by adding varying amounts of test materials followed by adenosine diphosphate (ADP, 20µM) to stirred human PRP. Specifically, the human PRP was incubated with the compound for 1-2 min. at 37°C prior to addition of the aggregating agent ADP. From full dose response curves the concentration (IC₅₀) to half-maximally inhibit platelet aggregation for test compounds was determined.

| TABLE OF ASSAY TEST RESULTS | | | |
|---|---|---|---|
| | GPIIbIIIa ELISA | αᵥβ₃ ELISA | PRP |
| Compound # (Example #) | (IC₅₀µM) | (IC₅₀µM) | (IC₅₀µM) |
| 4 | >100 | >200 | 120 |
| 5 | 50 | >100 | 67.1 |
| 6 | 0.8 | 0.9 | 4.9 |
| 7 | 1 | 50 | 4.9 |
| 8 | 0.1 | 0.3 | 0.26 |
| 9 | 0.02 | 0.1 | 0.17 |
| 11 | 0.1 | 0.2 | 0.85 |
| 12 | 0.6 | 3.0 | 5.7 |
| 13 | 2.5 | 0.5 | 3.3 |
| 14 | 2.5 | 5.0 | 1.0 |
| 15 | 0.08 | 3.0 | 0.22 |
| 16 | 5.0 | >100 | 49.3 |
| 17 | 0.1 | >100 | 1.1 |
| 18 | 10 | 40 | 122 |
| 20 | 0.075 | 15 | 0.55 |
| 107 (16) | 0.01 | 0.01 | 0.04 |
| 113(17) | 0.05 | 0.03 | 0.17 |
| 118(18) | 1.0 | | 3.7 |
| 126(19) | 0.05 | | 3.5 |
| 127(20) | | | 0.45 |
| 128(21) | 0.03 | | 0.21 |
| 131(22) | 0.02 | 2.5 | 0.16 |
| 137 (23) | 0.65 | 0.50 | 8.5 |
| 138 (24) | 0.06 | 0.02 | 0.18 |
| 139 (25) | 0.03 | 0.20 | 0.15 |
| 140(26) | 1.3 | 2.1 | 6.7 |
| 145 (27) | 0.05 | 0.40 | 0.23 |

As can be seen from the compounds listed in the Table of Assay Test Results, a number of compounds of the invention display potent inhibition of ligand binding to GPllb-IIIa and the vitronectin receptor, αᵥβ₃, as well as inhibition of ADP-induced human platelet aggregation with IC₅₀'s which are submicromolar and would be expected to display antiintegrin activities *in vivo.*

## Claims

1. A compound having the formula: 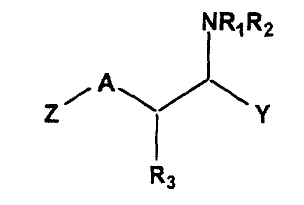 wherein:
Y is selected from the group consisting of -COOH, -PO₃H₂, -SO₃H and -COOR⁴; where R⁴ is selected from the group consisting of C₁₋₁₀alkyl, C₁₋₈alkylaryl, aryl-C₁₋₈alkyl, C₁₋₈alkyloxycarbonyloxy-C₁₋₈alkyl, aryloxycarbonyloxy-C₁₋₈alkyl, C₁₋₈alkyloxycarbonyloxyaryl, C₁₋₈alkylcarbonyloxy-C₁₋₈alkyl, arylcarbonyloxy-C₁₋₈alkyl and C₁₋₈alkylcarbonyloxyaryl;
A is selected from the group consisting of C₆₋₁₂alkyl, C₀₋₈alkyl-NR⁵-CO-C₀₋₈alkyl, C₀₋₈alkyl-CO-NR⁵-C₀₋₈alkyl, C₀₋₈alkyl-O-C₀₋₈alkyl, C₀₋₈alkyl-NR⁵-CO-C₁₋₈alkyl-NR⁵-CO-C₀₋₈alkyl, C₀₋₈alkyl-NR⁵-CO-C₁₋₈alkyl-CO-NR⁵-C₀₋₈alkyl, C₀₋₈alkyl-CO-NR⁵-C₁₋₈alkyl-NR⁵-CO-C₀₋₈alkyl, C₀₋₈alkyl-CO-NR⁵-C₀₋₈alkyl-CO-NR⁵-C₀₋₈alkyl, C₀₋₈alkyl-CO-C₁₋₈alkyl-CO-NR⁵-C₀₋₈alkyl, C₀₋₈alkyl-CO-C₀₋₈alkyl-NR⁵-CO-C₀₋₈alkyl, C₀₋₈alkyl-O-C₂₋₈alkyl-NR⁵-CO-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₀₋₈alkyl, C₀₋₈alkyl-S(Oₙ)-C₀₋₈alkyl, C₀₋₈alkyl-S-C₂₋₈alkyl-NR⁵-CO-C₀₋₈alkyl, C₀₋₈alkyl-S(Oₙ)-C₂₋₈alkyl-NR⁵-CO-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-CO-NR⁵-C₂₋₈alkyl-S-C₀₋₈alkyl, C₀₋₈alkyl-CO-NR⁵-C₂₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CS-O-C₀₋₈alkyl, C₀₋₈alkyl-NR⁵-C₀₋₈alkyl-CO-NR⁵-C₀₋₈alkyl, C₀₋₈alkyl-NR⁵-C₀₋₈alkyl-CS-NR⁵-C₀₋₈alkyl, C₀₋₈alkyl-O-C₀₋₈alkyl-CO₂-C₀₋₈alkyl, C₀₋₈alkyl-O-C₀₋₈alkyl-CS-O-C₀₋₈alkyl; C₀₋₈alkyl-SiR⁷R⁸-C₀₋₈alkyl, C₀₋₈alkyl-SiR⁷R⁸-C₀₋₈alkyl-NR⁶-CO-C₀₋₈alkyl, and C₀₋₈alkyl-SiR⁷R⁸-C₀₋₈alkyl-CO-NR⁶-C₀₋₈alkyl; where R⁵, R⁶, R⁷ and R⁸ are independently selected from the group consisting of H and C₁₋₆alkyl; and where n=1 or 2;
Z is selected from the group consisting of -NH-C(NR⁹R¹⁰)=NR¹¹, -NH-C(R⁹)=NR¹¹,-C(NR⁹R¹⁰)=NR¹¹ and piperidinyl; where R⁹, R¹⁰ and R¹¹ are independently selected from the group consisting of H, C₁₋₆alkyl, aryl-C₁₋₃alkyl and aryl; or where two of the R⁹, R¹⁰ or R¹¹ substituents form a cyclic ring containing (CH₂)ₚ, where p=2-5;
R¹ is H;
R² is selected from the group consisting of -SOₘ-aryl, -SOₘ-C₁₋₁₀alkyl and -SOₘ-heteroaryl, where m=1-2;
R³ is selected from the group consisting of H, C₁₋₈alkyl, aryl, C₁₋₈alkylaryl and heteroaryl;
and all pharmaceutically-acceptable stereoisomers, salts, hydrates, solvates and prodrug derivatives thereof.

2. The compound of Claim 1 wherein Y is selected from the group consisting of -COOH and -COOR⁴.

3. The compound of Claim 2 wherein Y is -COOH.

4. The compound of Claim 1 wherein R⁴ is C₁₋₁₀alkyl.

5. The compound of Claim 1 wherein A is selected from the group consisting of C₀₋₈alkyl-NR⁵-CO-C₀₋₈alkyl, C₀₋₈alkyl-CO-NR⁵-C₀₋₈alkyl, C₀₋₈alkyl-NR⁵-CO-C₁₋₈alkyl-NR⁵-CO-C₀₋₈alkyl, C₀₋₈alkyl-NR⁵-CO-C₁₋₈alkyl-CO-NR⁵-C₀₋₈alkyl, C₀₋₈alkyl-CO-NR⁵-C₁₋₈alkyl-NR⁵-CO-C₀₋₈alkyl, C₀₋₈alkyl-CO-NR⁵-C₀₋₈alkyl-CO-NR⁵-C₀₋₈alkyl, C₀₋₈alkyl-CO-C₁₋₈alkyl-CO-NR⁵-C₀₋₈alkyl, C₀₋₈alkyl-O-C₂₋₈alkyl-NR⁵-CO-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₂₋₈alkyl-NR⁵-CO-C₀₋₈alkyl, C₀₋₈alkyl-S(Oₙ)-C₂₋₈alkyl-NR⁵-CO-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-CO-NR⁵-C₂₋₈alkyl-S-C₀₋₈alkyl, C₀₋₈alkyl-CO-NR⁵-C₂₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CS-O-C₀₋₈alkyl, C₀₋₈alkyl-NR⁵-C₀₋₈alkyl-CO-NR⁵-C₀₋₈alkyl, C₀₋₈alkyl-NR⁵-C₀₋₈alkyl-CS-NR⁵-C₀₋₈alkyl, C₀₋₈alkyl-O-C₀₋₈alkyl-CO₂-C₀₋₈alkyl, C₀₋₈alkyl-O-C₀₋₈alkyl-CS-O-C₀₋₈alkyl; C₀₋₈alkyl-SiR⁷R⁸-C₀₋₈alkyl, C₀₋₈alkyl-SiR⁷R⁸-C₀₋₈alkyl-NR⁶-CO-C₀₋₈alkyl and C₀₋₈alkyl-SiR⁷R⁸-C₀₋₈alkyl-CO-NR⁶-C₀₋₈alkyl.

6. The compound of Claim 5 wherein A is selected from the group consisting of C₀₋₈alkyl-CO-NR⁵-C₀₋₈alkyl, C₀₋₈alkyl-O-C₀₋₈alkyl-CO-NR⁵-C₀₋₈alkyl, C₀₋₈alkyl-O-C₂₋₈alkyl-O-C₀₋₈alkyl-CO-NR⁵-C₀₋₈alkyl, C₀₋₈alkyl-S-C₀₋₈alkyl-CO-NR⁵-C₀₋₈alkyl, C₀₋₈alkyl-S(Oₙ)-C₁₋₈alkyl-CO-NR⁵-C₀₋₈alkyl, C₀₋₈alkyl-NR⁵-CO-C₀₋₈alkyl-S-C₀₋₈alkyl, C₀₋₈alkyl-NR⁵-CO-C₁₋₈alkyl-S(Oₙ)-C₀₋₈alkyl, C₀₋₈alkyl-NR⁵-C₀₋₈alkyl-CO₂-C₀₋₈alkyl, C₀₋₈alkyl-NR⁵-C₀₋₈alkyl-CO-NR⁵-C₀₋₈alkyl, -C₀₋₈alkyl-O-C₀₋₈alkyl-CO₂-C₀₋₈alkyl.

7. The compound of Claim 1 wherein Z is -NH-C(NR⁹R¹⁰)=NR¹¹.

8. The compound of Claim 7 wherein R⁹, R¹⁰ and R¹¹ are independently selected from the group consisting of H and C₁₋₆alkyl.

9. The compound of Claim 1 wherein R² is selected from the group consisting of -SO₂-aryl and -SO₂-C₁₋₁₀alkyl.

10. The compound of Claim 9 wherein R² is -SO₂-aryl.

11. The compound of Claim 1 wherein R³ is selected from the group consisting of H and C₁₋₈alkyl.

12. The compound of Claim 11 wherein R³ is H.

13. The compound of claim 1, having an IC₅₀ of less than about 200 nM.

14. A compound selected from a group consisting of:

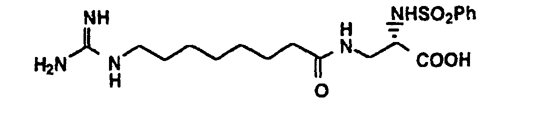

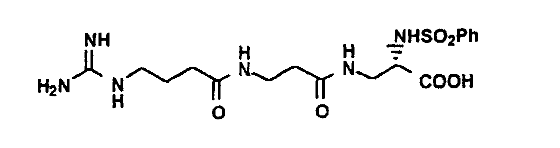

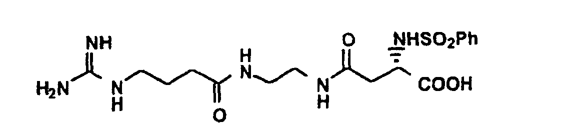

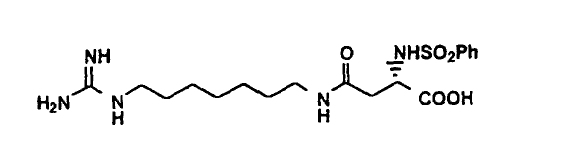

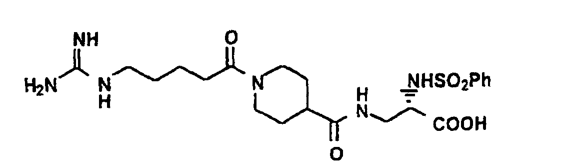

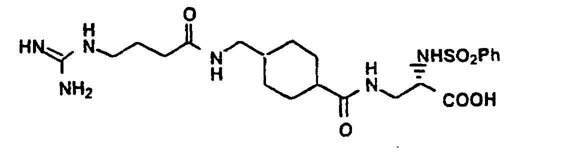

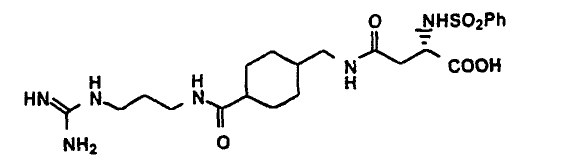

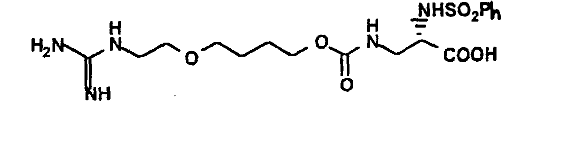

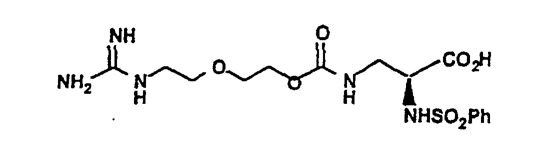

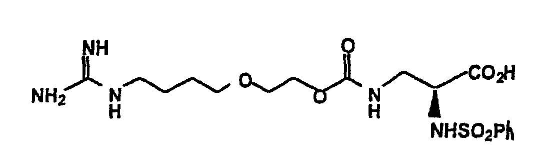

15. A pharmaceutical composition for preventing or treating a condition in a mammal **characterized by** undesired thrombosis comprising a therapeutically-acceptable carrier and a therapeutically-effective amount of a compound of Claim 1.

16. Use of a compound as defined in Claim 1 for the manufacture of a medicament for preventing or treating a condition in a mammal **characterized by** undesired thrombosis.

17. Use according to claim 16, wherein the condition is selected from the group consisting of: acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, transient ischemic attacks, deep venous thrombosis, pulmonary embolus, coagulopathy, disseminated intravascular coagulation, thrombotic thrombocytopenic purpura, thromboangiitis obliterans, thrombotic disease associated with heparin-induced thrombocytopenia, thrombotic complications associated with extracorporeal circulation, thrombotic complications associated with instrumentation such as cardiac or other intravascular catheterization, intra-aortic balloon pump, coronary stent or cardiac valve, conditions requiring the fitting of prosthetic devices, vascularization of solid tumors and retinopathy.

18. A process of making an ether linked amide having the general formula:
**Z-(L)-Q**_{**1**}
wherein Q₁ is 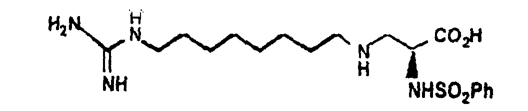 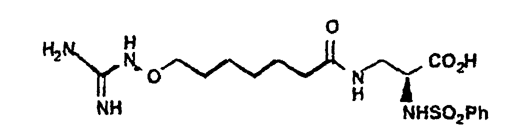 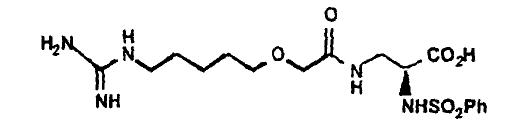 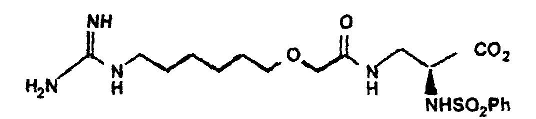 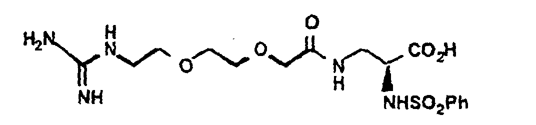 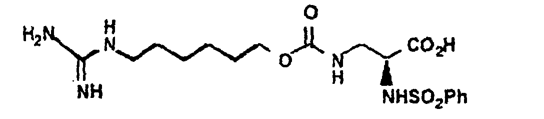 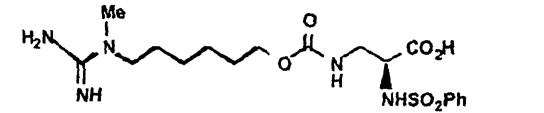 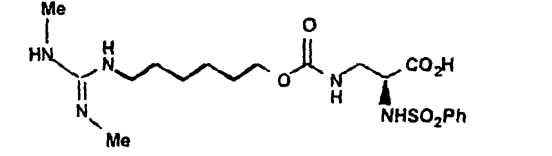 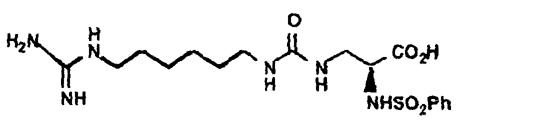 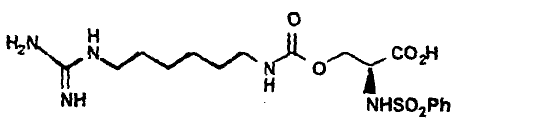 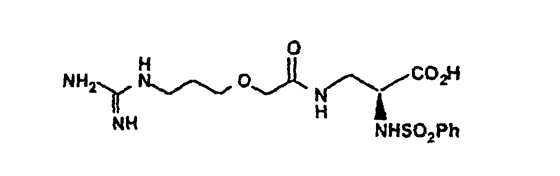 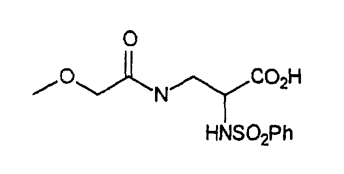
Z is selected from the group consisting of -NH-C(R⁹)=NR¹¹, -C(NR⁹R¹⁰)=NR¹¹ and piperidinyl; where R⁹, R¹⁰ and R¹¹ are independently selected from the group consisting of H, C₁₋₆alkyl, aryl-C₁₋₃ alkyl and aryl; or where two of the R⁹, R¹⁰ or R¹¹ substituents form a cyclic ring containing (CH₂)ₚ, where p=2-5;
L is a linking group selected from the group consisting of a bond, -O-, a divalent group derived from a C₁-C₈ alkane, and a divalent alkoxy group of the general formula -O-C₁-C₈-;
wherein the process comprises the sequential steps of
a) reacting an N-protected amino alcohol in the presence of a catalytic amount of rhodium catalyst with an alkyl diazoacetate to give an alpha alkoxy ester;
b) saponifying the reaction product of step (a) with a metal hydroxide to give a carboxylic acid;
c) reacting the product of step (b) with an amine and a carbodiamide dehydrating agent to give said ether linked amide.

19. A process of making an ether linked carbamate having the general formula
**Z-(L)-Q**_{**2**}
wherein
Q₂ is 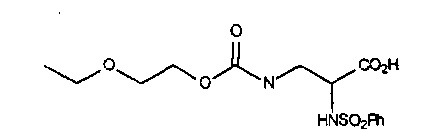
Z is selected from the group consisting of -NH-C(R⁹)=NR¹¹, -C(NR⁹R¹⁰)=NR¹¹ and piperidinyl; where R⁹, R¹⁰ and R¹¹ are independently selected from the group consisting of H, C₁₋₆ alkyl, aryl-C₁₋₃ alkyl and aryl; or where two of the R⁹, R¹⁰ or R¹¹ substituents form a cyclic ring containing (CH₂)ₚ, where p=2-5;
L is a linking group selected from the group consisting of a bond, -O-, a divalent group derived from a C₁-C₈ alkane, and a divalent alkoxy group of the general formula-O-C₁-C₈-;
wherein the process comprises the sequential steps of
a) reacting an N-protected amino alcohol in the presence of a catalytic amount of rhodium catalyst with an alkyl diazoacetate to give an alpha alkoxy ester;
b) saponifying the reaction product of step (a) with a metal hydroxide to give a carboxylic acid;
c) reducing the reaction of step (b) to give an alcohol; and
d) reacting the alcohol reaction product of step (c) first with phosgene and then with amine to give said ether linked carbamate.

20. The compound of Claim 1 for use in preventing or treating a condition in a mammal **characterized by** undesired thrombosis.

21. The compound of Claim 1 for use in preventing or treating a condition selected from the group consisting of: acute coronary syndrome, myocardial infarction, unstable angina, refractory angina, occlusive coronary thrombus occurring post-thrombolytic therapy or post-coronary angioplasty, a thrombotically mediated cerebrovascular syndrome, embolic stroke, thrombotic stroke, transient ischemic attacks, deep venous thrombosis, pulmonary embolus, coagulopathy, disseminated intravascular coagulation, thrombotic thrombocytopenic purpura, thromboangiitis obliterans, thrombotic disease associated with heparin-induced thrombocytopenia, thrombotic complications associated with extracorporeal circulation, thrombotic complications associated with instrumentation such as cardiac or other intravascular catheterization, intra-aortic balloon pump, coronary stent or cardiac valve, conditions requiring the fitting of prosthetic devices, vascularization of solid tumors and retinopathy.

## Patentansprüche

1. Verbindung mit der Formel: 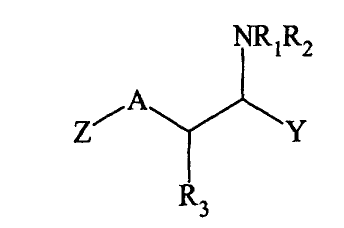 wobei:
Y ausgewählt ist aus der Gruppe bestehend aus -COOH, -PO₃H₂, -SO₃H und -COOR⁴; wobei R⁴ ausgewählt ist aus der Gruppe bestehend aus C₁₋₁₀Alkyl, C₁₋₈Alkylaryl, Aryl-C₁₋₈Alkyl, C₁₋₈Alkyloxycarbonyloxy-C₁₋₈Alkyl, Aryloxycarbonyloxy-C₁₋₈Alkyl, C₁₋₈Alkyloxycarbonyloxyaryl, C₁₋₈Alkylcarbonyloxy-C₁₋₈Alkyl, Arylcarbonyloxy-C₁₋₈Alkyl und C₁₋₈Alkylcarbonyloxyaryl;
A ausgewählt ist aus der Gruppe bestehend aus C₆₋₁₂Alkyl, C₀₋₈Alkyl-NR⁵-CO-C₀₋₈Alkyl, C₀₋₈Alkyl-CO-NR⁵-C₀₋₈Alkyl, C₀₋₈Alkyl-O-C₀₋₈Alkyl, C₀₋₈Alkyl-NR⁵-CO-C₁₋₈Alkyl-NR⁵-CO-C₀₋₈Alkyl, C₀₋₈Alkyl-NR⁵-CO-C₁₋₈Alkyl-CO-NR⁵-C₀₋₈Alkyl, C₀₋₈Alkyl-CO-NR⁵-C₁₋₈Alkyl-NR⁵-CO-C₀₋₈Alkyl, C₀₋₈Alkyl-CO-NR⁵-C₀₋₈Alkyl-CO-NR⁵-C₀₋₈Alkyl, C₀₋₈Alkyl-CO-C₁₋₈Alkyl-CO-NR⁵-C₀₋₈Alkyl, C₀₋₈Alkyl-CO-C₀₋₈Alkyl-NR⁵-CO-C₀₋₈Alkyl, C₀₋₈Alkyl-O-C₂₋₈Alkyl-NR⁵-CO-C₀₋₈Alkyl, C₀₋₈Alkyl-O-C₀₋₈Alkyl-CO-NR⁵-C₀₋₈Alkyl, C₀₋₈Alkyl-O-C₂₋₈Alkyl-O-C₀₋₈Alkyl-CO-NR⁵-C₀₋₈Alkyl, C₀₋₈Alkyl-S-C₀₋₈Alkyl, C₀₋₈Alkyl-S(Oₙ)-C₀₋₈Alkyl, C₀₋₈Alkyl-S-C₂₋₈Alkyl-NR⁵-CO-C₀₋₈Alkyl, C₀₋₈Alkyl-S(Oₙ)-C₂₋₈Alkyl-NR⁵-CO-C₀₋₈Alkyl, C₀₋₈Alkyl-S-C₀₋₈Alkyl-CO-NR⁵-C₀₋₈Alkyl, C₀₋₈Alkyl-S(Oₙ)-C₁₋₈Alkyl-CO-NR⁵-C₀₋₈Alkyl, C₀₋₈Alkyl-NR⁵-CO-C₀₋₈Alkyl-S-C₀₋₈Alkyl, C₀₋₈Alkyl-NR⁵-CO-C₁₋₈Alkyl-S(Oₙ)-C₀₋₈Alkyl, C₀₋₈Alkyl-CO-NR⁵-C₂₋₈Alkyl-S-C₀₋₈Alkyl, C₀₋₈Alkyl-CO-NR⁵-C₂₋₈Alkyl-S(Oₙ)-C₀₋₈Alkyl, C₀₋₈Alkyl-NR⁵-C₀₋₈Alkyl-CO₂-C₀₋₈Alkyl, C₀₋₈Alkyl-NR⁵-C₀₋₈Alkyl-CS-O-C₀₋₈Alkyl, C₀₋₈Alkyl-NR⁵-C₀₋₈Alkyl-CO-NR⁵-C₀₋₈Alkyl, C₀₋₈Alkyl-NR⁵-C₀₋₈Alkyl-CS-NR⁵-C₀₋₈Alkyl, C₀₋₈Alkyl-O-C₀₋₈Alkyl-CO₂-C₀₋₈Alkyl, C₀₋₈Alkyl-O-C₀₋₈Alkyl-CS-O-C₀₋₈Alkyl, C₀₋₈Alkyl-SiR⁷R⁸-C₀₋₈Alkyl, C₀₋₈Alkyl-SiR⁷R⁸-C₀₋₈Alkyl-NR⁶-CO-C₀₋₈Alkyl and C₀₋₈Alkyl-SiR⁷R⁸-C₀₋₈Alkyl-CO-NR⁶-C₀₋₈Alkyl; wobei R⁵, R⁶, R⁷ und R⁸ unabhängig voneinander ausgewählt sind aus der Gruppe bestehend aus H und C₁₋₆Alkyl; und wobei n = 1 oder 2 gilt;
Z ausgewählt ist aus der Gruppe bestehend aus -NH-C(NR⁹R¹⁰)=NR¹¹, -NH-C(R⁹)=NR¹¹, -C(NR⁹R¹⁰)=NR¹¹ und Piperidinyl; wobei R⁹, R¹⁰ und R¹¹ unabhängig voneinander ausgewählt sind aus der Gruppe bestehend aus H, C₁₋₆Alkyl, Aryl-C₁₋₃Alkyl und Aryl, oder wobei zwei der R⁹-, R¹⁰- oder R¹¹-Substituenten einen (CH₂)ₚ enthaltenden cyclischen Ring bilden, wobei p = 2-5 gilt;
R¹ ist H;
R₂ ausgewählt ist aus der Gruppe bestehend aus -SOₘ-Aryl, -SOₘ-C₁₋₁₀Alkyl und -SOₘ-Heteroaryl, wobei m = 1-2 ist;
R₃ ausgewählt ist aus der Gruppe bestehend aus H, C₁₋₈Alkyl, Aryl, C₁₋₈Alkylaryl und Heteroaryl;
und alle deren pharmazeutisch akzeptablen Stereoisomere, Salze, Hydrate, Solvate und Prodrug-Derivate.

2. Verbindung gemäß Anspruch 1, wobei Y ausgewählt ist aus der Gruppe bestehend aus -COOH und -COOR⁴.

3. Verbindung gemäß Anspruch 2, wobei Y -COOH ist.

4. Verbindung gemäß Anspruch 1, wobei R⁴ C₁₋₁₀Alkyl ist.

5. Verbindung gemäß Anspruch 1, wobei A ausgewählt ist aus der Gruppe bestehend aus C₀₋₈Alkyl-NR⁵-CO-C₀₋₈Alkyl, C₀₋₈Alkyl-CO-NR⁵-C₀₋₈Alkyl, C₀₋₈Alkyl-NR⁵-CO-C₁₋₈AlkylNR⁵-CO-C₀₋₈Alkyl, C₀₋₈Alkyl-NR⁵-CO-C₁₋₈Alkyl-CO-NR⁵-C₀₋₈Alkyl, C₀₋₈Alkyl-CO-NR⁵-C₁₋₈Alkyl-NR⁵-CO-C₀₋₈Alkyl, C₀₋₈Alkyl-CO-NR⁵-C₀₋₈Alkyl-CO-NR⁵-C₀₋₈Alkyl, C₀₋₈Alkyl-CO-C₁₋₈Alkyl-CO-NR⁵-C₀₋₈Alkyl, C₀₋₈Alkyl-O-C₂₋₈Alkyl-NR⁵-CO-C₀₋₈Alkyl, C₀₋₈Alkyl-O-C₀₋₈Alkyl-CO-NR⁵-C₀₋₈Alkyl, C₀₋₈ Alkyl-O-C₂₋₈Alkyl-O-C₀₋₈Alkyl-CO-NR⁵-C₀₋₈Alkyl, C₀₋₈Alkyl-S-C₂₋₈Alkyl-NR⁵-CO-C₀₋₈Alkyl, C₀₋₈Alkyl-S(Oₙ)-C₂₋₈Alkyl-NR⁵-CO-C₀₋₈Alkyl, C₀₋₈Alkyl-S-C₀₋₈ Alkyl-CO-NR⁵-C₀₋₈Alkyl, C₀₋₈Alkyl-S(Oₙ)-C₁₋₈Alkyl-CO-NR⁵-C₀₋₈Alkyl, C₀₋₈Alkyl-NR⁵-CO-C₀₋₈Alkyl-S-C₀₋₈Alkyl, C₀₋₈Alkyl-NR⁵-CO-C₁₋₈Alkyl-S(Oₙ)-C₀₋₈Alkyl, C₀₋₈Alkyl-CO-NR⁵-C₂₋₈Alkyl-S-C₀₋₈Alkyl, C₀₋₈Alkyl-CO-NR⁵-C₂₋₈Alkyl-S(Oₙ)-C₀₋₈Alkyl, C₀₋₈Alkyl-NR⁵-C₀₋₈Alkyl-CO₂-C₀₋₈Alkyl, C₀₋₈Alkyl-NR⁵-C₀₋₈Alkyl-CS-O-C₀₋₈Alkyl, C₀₋₈Alkyl-NR⁵-C₀₋₈Alkyl-CO-NR⁵-C₀₋₈Alkyl, C₀₋₈Alkyl-NR⁵-C₀₋₈Alkyl-CS-NR⁵-C₀₋₈Alkyl, C₀₋₈Alkyl-O-C₀₋₈Alkyl-CO₂-C₀₋₈Alkyl, C₀₋₈Alkyl-O-C₀₋₈Alkyl-CS-O-C₀₋₈Alkyl, C₀₋₈Alkyl-SiR⁷R⁸-C₀₋₈Alkyl, C₀₋₈Alkyl-SiR⁷R⁸-C₀₋₈Alkyl-NR⁶-CO-C₀₋₈Alkyl und C₀₋₈Alkyl-SiR⁷R⁸-C₀₋₈Alkyl-CO-NR⁶-C₀₋₈Alkyl.

6. Verbindung gemäß Anspruch 5, wobei A ausgewählt ist aus der Gruppe bestehend aus C₀₋₈Alkyl-CO-NR⁵-C₀₋₈Alkyl, C₀₋₈Alkyl-O-C₀₋₈Alkyl-CO-NR⁵-C₀₋₈Alkyl, C₀₋₈Alkyl-O-C₂₋₈Alkyl-O-C₀₋₈Alkyl-CO-NR⁵-C₀₋₈Alkyl, C₀₋₈Alkyl-S-C₀₋₈Alkyl-CO-NR⁵-C₀₋₈Alkyl, C₀₋₈Alkyl-S(Oₙ)-C₁₋₈Alkyl-CO-NR⁵-C₀₋₈Alkyl, C₀₋₈ Alkyl-NR⁵-CO-C₀₋₈Alkyl-S-C₀₋₈Alkyl, C₀₋₈Alkyl-NR⁵-CO-C₁₋₈Alkyl-S(Oₙ)-C₀₋₈Alkyl, C₀₋₈Alkyl-NR⁵-C₀₋₈Alkyl-CO₂-C₀₋₈Alkyl, C₀₋₈Alkyl-NR⁵-C₀₋₈Alkyl-CO-NR⁵-C₀₋₈Alkyl, C₀₋₈Alkyl-O-C₀₋₈Alkyl-CO₂-C₀₋₈Alkyl.

7. Verbindung gemäß Anspruch 1, wobei Z -NH-C(NR⁹R¹⁰)=NR¹¹ ist.

8. Verbindung gemäß Anspruch 7, wobei R⁹, R¹⁰ und R¹¹ unabhängig voneinander ausgewählt sind aus der Gruppe bestehend aus H und C₁₋₆Alkyl.

9. Verbindung gemäß Anspruch 1, wobei R² ausgewählt ist aus der Gruppe bestehend aus -SO₂-Aryl und -SO₂-C₁₋₁₀Alkyl.

10. Verbindung gemäß Anspruch 9, wobei R² -SO₂-Aryl ist.

11. Verbindung gemäß Anspruch 1, wobei R³ ausgewählt ist aus der Gruppe bestehend aus H und C₁₋₈Alkyl.

12. Verbindung gemäß Anspruch 11 wobei R³ H ist.

13. Verbindung gemäß Anspruch 1 mit einem IC₅₀-Wert von weniger als etwa 200 nM.

14. Verbindung ausgewählt aus der Gruppe bestehend aus:

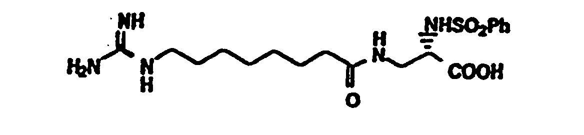

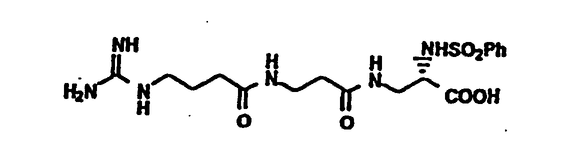

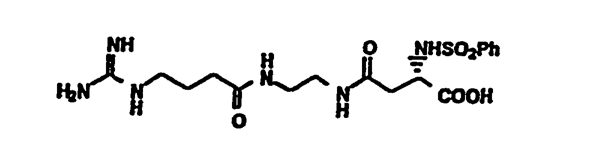

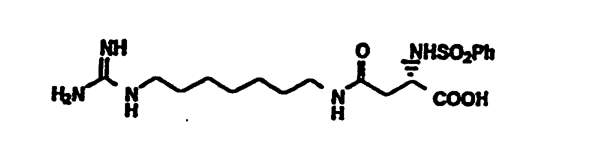

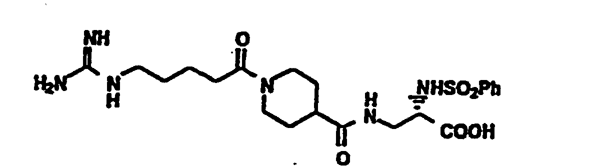

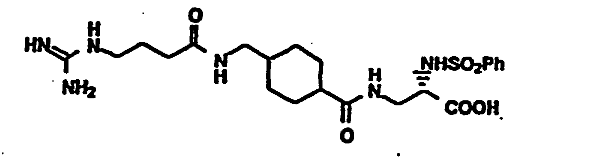

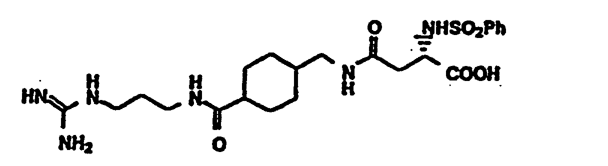

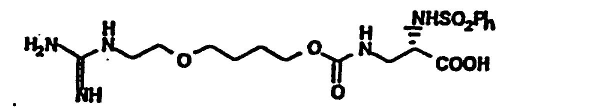

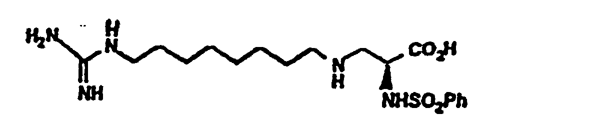

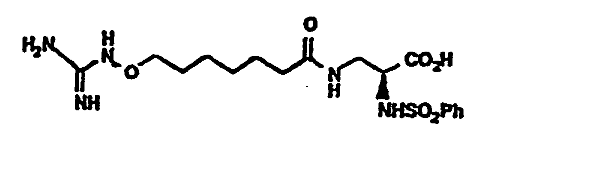

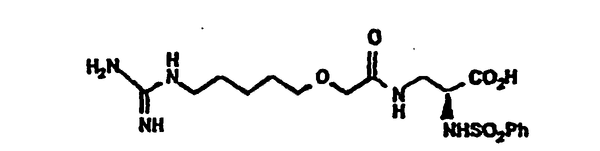

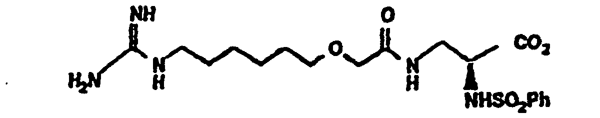

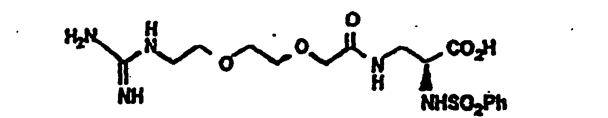

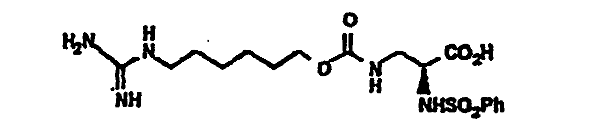

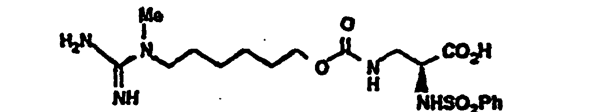

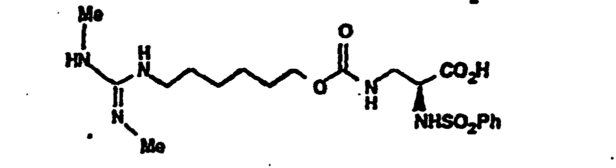

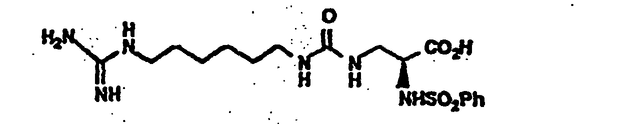

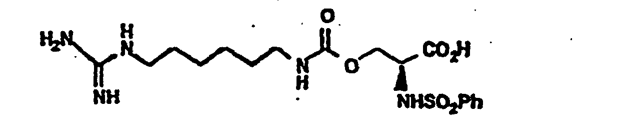

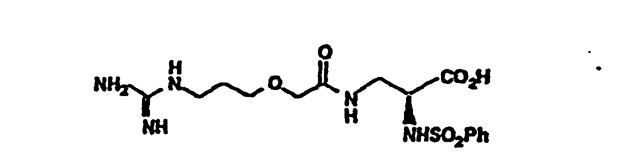

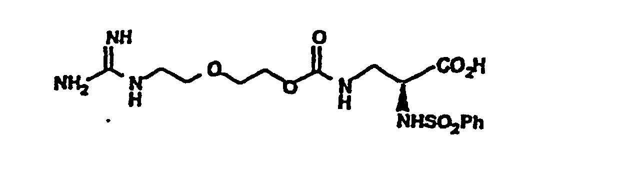

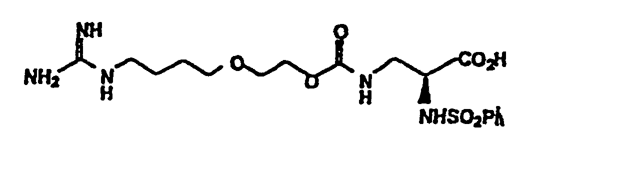

15. Pharmazeutische Zusammensetzung zur Verhinderung oder Behandlung eines Zustandes in einem Säugetier, der durch unerwünschte Thrombose charakterisiert ist, umfassend einen therapeutisch akzeptablen Träger und eine therapeutisch effektive Menge einer Verbindung gemäß Anspruch 1.

16. Verwendung einer Verbindung wie in Anspruch 1 definiert für die Herstellung eines Arzneimittels zur Verhinderung oder Behandlung eines Zustandes in einem Säugetier, der durch unerwünschte Thrombose charakterisiert ist.

17. Verwendung gemäß Anspruch 16, wobei der Zustand ausgewählt ist aus der Gruppe bestehend aus: Akutem Koronarsyndrom, myokardialer Infarktierung, instabiler Angina, refraktärer Angina, okklusivem koronarem Thrombus auftretend post-thrombolytischer Therapie oder post-koronarer Angioplastie, einem thrombotisch vermittelten zerebrovaskulären Syndrom, embolischem Schlaganfall, thrombotischem Schlaganfall, vorübergehenden ischämischen Anfällen, tiefe Venenthrombose, pulmonalem Embolus, Koagulopathie, disseminierte intravasale Koagulation, thrombotische thrombocytopenische Purpura, Thromboangiitis obliterans, mit Heparin-induzierter Thrombozytopenie assoziierter thrombotischer Krankheit, mit extrakorporaler Zirkulation assoziierte thrombotische Komplikationen, mit Instrumentierung wie Herzkatheterisierung oder anderer intravasaler Katherterisierung, intraaortaler Ballonpumpe, koronarem Stent oder einer Herzklappe assozierte thrombotische Komplikationen, das Anpassen von prothetischen Vorrichtungen erfordernde Zustände, Vaskularisierung von festen Tumoren und Retinopathie.

18. Verfahren zur Herstellung eines mit einem Ether verbundenen Säureamids mit der allgemeinen Formel:
Z-(L)-Q₁
Wobei
Q₁ 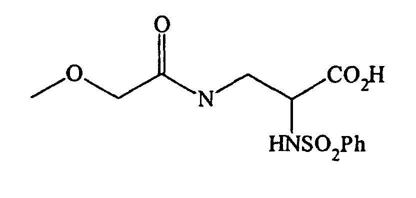 ist,
Z ausgewählt ist aus der Gruppe bestehend aus -NH-C(R⁹)=NR¹¹, -C(NR⁹R¹⁰)=NR¹¹ und Piperidinyl; wobei R⁹, R¹⁰ und R¹¹ unabhängig voneinander ausgewählt sind aus der Gruppe bestehend aus H, C₁₋₆Alkyl, Aryl-C₁₋₃Alkyl und Aryl, oder wobei zwei der R⁹-, R¹⁰- oder R¹¹-Substituenten einen (CH₂)ₚ enthaltenden cyclischen Ring bilden, wobei p = 2-5 gilt;
L eine Verbindungsgruppe ist ausgewählt aus der Gruppe bestehend aus einer Bindung, -O-, einer von einem C₁₋₈-Alkan abgeleiteten, divalenten Gruppe und einer divalenten Alkoxygruppe der allgemeinen Formel -O-C₁-C₈-;
wobei das Verfahren die aufeinanderfolgenden Schritte
a) zur Reaktion bringen eines N-geschützen Aminoalkohols in der Gegenwart einer katalytischen Menge Rhodiumkatalysators mit einem Alkyldiazoacetat, um einen Alpha-Alkoxyester zu erhalten;
b) Verseifen des Reaktionsproduktes aus Schritt (a) mit einem Metallhydroxid, um eine Carbonsäure zu erhalten;
c) zur Reaktion bringen des Produktes aus Schritt (b) mit einem Amin und einem Carbodiamid-Dehydratisierungsmittel, um das mit einem Ether verbundene Säureamid zu erhalten.

19. Verfahren zur Herstellung eines mit einem Ether verbundenen Carbamats mit der allgemeinen Formel
Z-(L)-Q₂
wobei
Q₂ 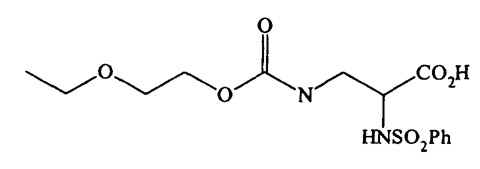 ist;
Z ausgewählt ist aus der Gruppe bestehend aus -NH-C(R⁹)=NR¹¹, -C(NR⁹R¹⁰)=NR¹¹ und Piperidinyl; wobei R⁹, R¹⁰ und R¹¹ unabhängig voneinander ausgewählt sind aus der Gruppe bestehend aus H, C₁₋₆Alkyl, Aryl-C₁₋₃Alkyl und Aryl, oder wobei zwei der R⁹-, R¹⁰- oder R¹¹-Substituenten einen (CH₂)ₚ enthaltenden cyclischen Ring bilden, wobei p = 2-5 gilt;
L eine Verbindungsgruppe ist ausgewählt aus der Gruppe bestehend aus einer Bindung, -O-, einer von einem C₁₋₈-Alkan abgeleiteten, divalenten Gruppe und einer divalenten Alkoxygruppe der allgemeinen Formel -O-C₁-C₈-;
wobei das Verfahren die aufeinanderfolgenden Schritte
a) zur Reaktion bringen eines N-geschützen Aminoalkohols in der Gegenwart einer katalytischen Menge Rhodiumkatalysators mit einem Alkyldiazoacetat, um einen Alpha-Alkoxyester zu erhalten;
b) Verseifen des Reaktionsproduktes aus Schritt (a) mit einem Metallhydroxid, um eine Carbonsäure zu erhalten;
c) Reduzieren der Reaktion von Schritt (b), um einen Alkohol zu erhalten,
d) zur Reaktion bringen des Alkohol-Reaktionsproduktes des Schrittes (c) zuerst mit Phosgen und dann mit Amin um das mit einem Ether verbundene Carbamat zu erhalten.

20. Verbindung gemäß Anspruch 1 zur Verwendung zur Verhinderung oder Behandlung eines Zustandes in einem Säugetier, der durch unerwünschte Thrombose charakterisiert ist.

21. Verbindung gemäß Anspruch 1 zur Verwendung zur Verhinderung oder Behandlung eines Zustands ausgewählt aus der Gruppe bestehend aus: Akutem Koronarsyndrom, myokardialer Infarktierung, instabiler Angina, refraktärer Angina, okklusivem koronarem Thrombus auftretend post-thrombolytischer Therapie oder post-koronarer Angioplastie, einem thrombotisch vermittelten zerebrovaskulären Syndrom, embolischem Schlaganfall, thrombotischem Schlaganfall, vorübergehenden ischämischen Anfällen, tiefe Venenthrombose, pulmonalem Embolus, Koagulopathie, disseminierte intravasale Koagulation, thrombotische thrombocytopenische Purpura, Thromboangiitis obliterans, mit Heparin-induzierter Thrombozytopenie assoziierter thrombotischer Krankheit, mit extrakorporaler Zirkulation assoziierte thrombotische Komplikationen, mit Instrumentierung wie Herzkatheterisierung oder anderer intravasaler Katherterisierung, intraaortaler Ballonpumpe, koronarem Stent oder einer Herzklappe assozierte thrombotische Komplikationen, das Anpassen von prothetischen Vorrichtungen erfordernde Zustände, Vaskularisierung von festen Tumoren und Retinopathie.

## Revendications

1. Composé de formule : 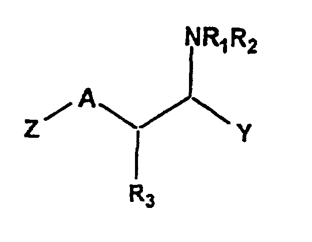 dans laquelle :
Y est sélectionné dans le groupe consistant en -COOH, -PO₃H₂, -SO₃H et -COOR⁴ ;
où R⁴ est sélectionné dans le groupe consistant en alkyle en C₁₋₁₀, alkyl(en C₁₋₈)aryle, arylalkyle en C₁₋₈, alkyl(en C₁₋₈)oxycarbonyloxy-alkyle en C₁₋₈, aryloxycarbonyloxy-alkyle en C₁₋₈, alkyl(en C₁₋₈)oxycarbonyloxyaryle, alkyl(en C₁₋₈)carbonyloxy-alkyle en C₁₋₈, arylcarbonyloxyalkyle en C₁₋₈ et alkyl(en C₁₋₈)carbonyloxyaryle :
A est sélectionné dans le groupe consistant en alkyle en C₆₋₁₂, alkyl(en C₀₋₈)-NR⁵-CO-alkyle en C₀₋₈, alkyl(en C₀₋₈)-CO-NR⁵-alkyle en C₀₋₈, alkyl(en C₀₋₈)-O-alkyl(en C₀₋₈), alkyl(en C₀₋₈)-NR⁵-CO-alkyl(en C₁₋₈)-NR⁵-CO-alkyle en C₀₋₈, alkyl(en C₀₋₈)-NR⁵-CO-alkyl(en C₁₋₈)-CO-NR⁵-alkyle en C₀₋₈, alkyl(en C₀₋₈)-CO-NR⁵-alkyl(en C₁₋₈)-NR⁵-CO-alkyle en C₀₋₈, alkyl(en C₀₋₈)-CO-NR⁵-alkyl(en C₀₋₈)-CO-NR⁵-alkyle en C₀₋₈, alkyl(en C₀₋₈)-CO-alkyl(en C₀₋₈)-CO-NR⁵-alkyle en C₀₋₈, alkyl(en C₀₋₈)-CO-alkyl(en C₀₋₈)-NR⁵-CO-alkyle en C₀₋₈, alkyl(en C₀₋₈)-O-alkyl(en C₂₋₈)-NR⁵-CO-alkyle en C₀₋₈, alkyl(en C₀₋₈)-O-alkyl(en C₀₋₈)-CO-NR⁵-alkyle en C₀₋₈, alkyl(en C₀₋₈)-O-alkyl(en C₂₋₈)-O-alkyl(en C₀₋₈)-CO-NR⁵-alkyle en C₀₋₈, alkyl(en C₀₋₈)-S-alkyle en C₀₋₈, alkyl(en C₀₋₈)-S(Oₙ)alkyle en C₀₋₈, alkyl(en C₀₋₈)-S-alkyl(en C₂₋₈)-NR⁵-CO-alkyle en C₀₋₈, alkyl(en C₀₋₈)-S(Oₙ) alkyl(en C₂₋₈)-NR⁵-CO-alkyle en C₀₋₈, alkyl(en C₀₋₈)-S-alkyl(en C₀₋₈)-CO-NR⁵-alkyle en C₀₋₈, alkyl(en C₀₋₈)-S(Oₙ)-alkyle(en C₁₋₈)-CO-NR⁵-alkyle en C₀₋₈, alkyl(en C₀₋₈)-NR⁵-CO-alkyl(en C₀₋₈)-S-alkyle en C₀₋₈, alkyl(en C₀₋₈)-NR⁵-CO-alkyl(en C₁₋₈)-S(Oₙ)-alkyle en C₀₋₈, alkyl(en C₀₋₈)-CO-NR⁵-alkyl(en C₂₋₈)-S-alkyle en C₀₋₈, alkyl(en C₀₋₈)-CO-NR⁵-alkyl(en C₂₋₈)-S(Oₙ)-alkyle en C₀₋₈, alkyl(en C₀₋₈)-NR⁵-alkyl(en C₀₋₈)-CO₂-alkyle en C₀₋₈, alkyl(en C₀₋₈)-NR⁵-alkyl(en C₀₋₈)-CS-O-alkyle en C₀₋₈), alkyl(en C₀₋₈)-NR⁵-alkyl(en C₀₋₈)-CO-NR⁵-alkyle en C₀₋₈, alkyl(en C₀₋₈)-NR⁵-alkyl(en C₀₋₈)-CS-NR⁵-alkyle en C₀₋₈, alkyl(en C₀₋₈)-O-alkyl(en C₀₋₈)-CO₂-alkyle en C₀₋₈, alkyl(en C₀₋₈)-O-alkyl(en C₀₋₈)-CS-O-alkyle en C₀₋₈, alkyl(en C₀₋₈)-SiR⁷R⁸-alkyle en C₀₋₈, alkyl(en C₀₋₈)-SiR⁷R⁸-alkyl(en C₀₋₈)-NR⁶-CO-alkyle en C₀₋₈) et alkyl(en C₀₋₈)-SiR⁷R⁸-alkyl(en C₀₋₈)-CO-NR⁶-alkyle en C₀₋₈, où R⁵, R⁶, R⁷ et R⁸ sont indépendamment sélectionnés parmi le groupe consistant en H et alkyle en C₁₋₆ ; et où n=1 ou 2 ;
Z est sélectionné dans le groupe consistant en -NH-C(NR⁹R¹⁰)=NR¹¹, -NH-C(R⁹)=NR¹¹, -C(NR⁹R¹⁰)=NR¹¹ et pipéridinyle ; où R⁹, R¹⁰ et R¹¹ sont indépendamment sélectionnés dans le groupe consistant en H, alkyle en C₁₋₆, aryl-alkyle en C₁₋₃ et aryle ; ou bien, où deux des substituants R⁹, R¹⁰ et R¹¹ forment un noyau cyclique contenant (CH₂)ₚ, où p = 2-5 ;
R¹ représente H ;
R² est sélectionné dans le groupe consistant en -SOₘ-aryle, -Soₘ-alkyle en C₁₋₁₀ et -SOₘ-hétéroaryle, où m = 1-2 ;
R³ est sélectionné dans le groupe consistant en H, alkyle en C₁₋₈, aryle, alkyl(en C₁₋₈)aryle et hétéroaryle ; et tous les stéréoisomères, sels, hydrates, solvates et dérivés pro-médicaments pharmaceutiquement acceptables d'un tel composé.

2. Composé selon la revendication 1, dans lequel Y est sélectionné dans le groupe consistant en -COOH et -COOR⁴.

3. Composé selon la revendication 2, dans lequel Y représente -COOH.

4. Composé selon la revendication 1 dans lequel R⁴ représente alkyle en C₁₋₁₀.

5. Composé selon la revendication 1, dans lequel A est sélectionné dans le groupe consistant en alkyl(en C₀₋₈)-NR⁵-CO-alkyle en C₀₋₈, alkyl(en C₀₋₈)-CO-NR⁵-alkyle en C₀₋₈, alkyl(en C₀₋₈)-NR⁵-CO-alkyl(en C₁₋₈)-NR⁵-CO-alkyle en C₀₋₈, alkyl(en C₀₋₈)-NR⁵-CO-alkyl(en C₁₋₈)-CO-NR⁵-alkyle en C₀₋₈, alkyl(en C₀₋₈)-CO-NR⁵-alkyl(en C₁₋₈)-NR⁵-CO-alkyle en C₀₋₈, alkyl(en C₀₋₈)-CO-NR⁵-alkyl(en C₀₋₈)-CO-NR⁵-alkyle en C₀₋₈, alkyl(en C₀₋₈)-CO-alkyl(en C₀₋₈)-CO-NR⁵-alkyle en C₀₋₈, alkyl(en C₀₋₈)-O-alkyl(en C₂₋₈)-NR⁵-CO-alkyle en C₀₋₈, alkyl(en C₀₋₈)-O-alkyl(en C₀₋₈)-CO-NR⁵-alkyle en C₀₋₈, alkyl(en C₀₋₈)-O-alkyl(en C₂₋₈)-O-alkyl(en C₀₋₈)-CO-NR⁵-alkyle en C₀₋₈, alkyl(en C₀₋₈)-S-alkyl(en C₂₋₈)-NR⁵-CO-alkyle en C₀₋₈, alkyl(en C₀₋₈)-S(Oₙ)alkyl(en C₂₋₈)-NR⁵-CO-alkyle en C₀₋₈, alkyl(en C₀₋₈)-S-alkyl(en C₀₋₈)-CO-NR⁵-alkyle en C₀₋₈, alkyl(en C₀₋₈)-S(Oₙ)-alkyl(en C₁₋₈)-CO-NR⁵-alkyle en C₀₋₈, alkyl(en C₀₋₈)-NR⁵-CO-alkyl(en C₀₋₈)-S-alkyle en C₀₋₈, alkyl(en C₀₋₈)-NR⁵-CO-alkyl(en C₁₋₈)-S(Oₙ)-alkyle en C₀₋₈, alkyl(en C₀₋₈)-CO-NR⁵-alkyl(en C₂₋₈)-S-alkyle en C₀₋₈, alkyl(en C₀₋₈)-CO-NR⁵-alkyl(en C₂₋₈)-S(Oₙ)-alkyle en C₀₋₈, alkyl(en C₀₋₈)-NR⁵-alkyl(en C₀₋₈)-CO₂-alkyle en C₀₋₈, alkyl(en C₀₋₈)-NR⁵-alkyl(en C₀₋₈)-CS-O-alkyle en C₀₋₈, alkyl(en C₀₋₈)-NR⁵-alkyl(en C₀₋₈)-CO-NR⁵-alkyle en C₀₋₈, alkyl(en C₀₋₈)-NR⁵-alkyl(en C₀₋₈)-CS-NR⁵-alkyle en C₀₋₈, alkyl(en C₀₋₈)-O-alkyl(en C₀₋₈)-CO₂-alkyle en C₀₋₈, alkyl(en C₀₋₈)-O-alkyl(en C₀₋₈)-CS-O-alkyle en C₀₋₈, alkyl(en C₀₋₈)-SiR⁷R⁸-alkyle en C₀₋₈, alkyl(en C₀₋₈)-SiR⁷R⁸-alkyl(en C₀₋₈)-NR⁶-CO-alkyle en C₀₋₈ et alkyl(en C₀₋₈)-SiR⁷R⁸-alkyl(en C₀₋₈)-CO-NR⁶-alkyle en C₀₋₈.

6. Composé selon la revendication 5, dans lequel A est sélectionné dans le groupe consistant en alkyl(en C₀₋₈)-CO-NR⁵-alkyle en C₀₋₈, alkyl(en C₀₋₈)-O-alkyl(en C₀₋₈)-CO-NR⁵-alkyle en C₀₋₈, alkyl(en C₀₋₈)-O-alkyl(en C₂₋₈)-O-alkyl(en C₀₋₈)-CO-NR⁵-alkyle en C₀₋₈, alkyl(en C₀₋₈)-S-alkyl(en C₀₋₈)-CO-NR⁵-alkyle en C₀₋₈, alkyl(en C₀₋₈)-S(Oₙ)-alkyl(en C₁₋₈)-CO-NR⁵-alkyle en C₀₋₈, alkyl(en C₀₋₈)-NR⁵-CO-alkyl(en C₀₋₈)-S-alkyle en C₀₋₈, alkyl(en C₀₋₈)-NR⁵-CO-alkyl(en C₁₋₈)-S(Oₙ)-alkyle en C₀₋₈, alkyl(en C₀₋₈)-NR⁵-alkyl(en C₀₋₈)-CO₂-alkyle en C₀₋₈, alkyl(en C₀₋₈)-NR⁵-alkyl(en C₀₋₈)-CO-NR⁵-alkyle en C₀₋₈, alkyl(en C₀₋₈)-O-alkyl(en C₀₋₈)-CO₂-alkyle en C₀₋₈.

7. Composé selon la revendication 1 dans lequel Z représente -NH-C(NR⁹R¹⁰)=NR¹¹.

8. Composé selon la revendication 7, dans lequel R⁹, R¹⁰ et R¹¹ sont indépendamment sélectionnés dans le groupe consistant en H et alkyle en C₁₋₆.

9. Composé selon la revendication 1, dans lequel R² est sélectionné dans le groupe consistant en -SO₂-aryle et -SO₂-alkyle en C₁₋₁₀.

10. Composé selon la revendication 9, dans lequel R² représente -SO₂-aryle.

11. Composé selon la revendication 1, dans lequel R³ est sélectionné dans le groupe consistant en H et alkyle en C₁₋₈.

12. Composé selon la revendication 11, dans lequel R³ représente H.

13. Composé selon la revendication 1 ayant une CI₅₀ inférieure à environ 200 nM.

14. Composé sélectionné dans le groupe consistant en :

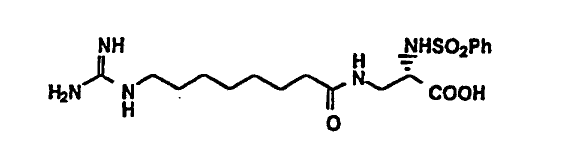

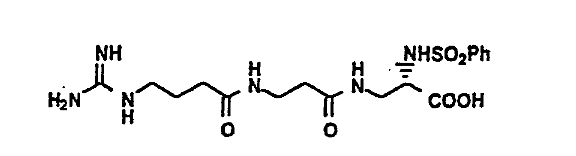

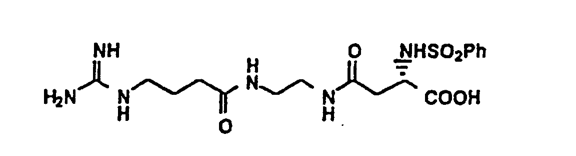

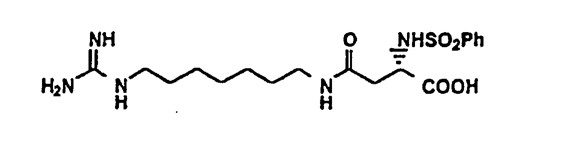

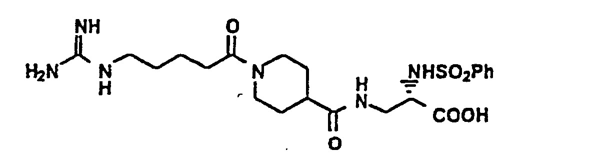

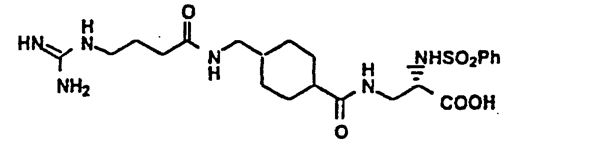

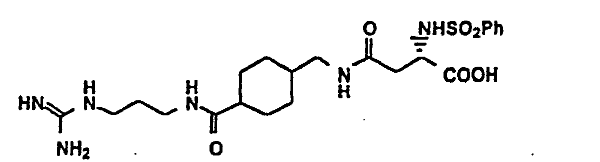

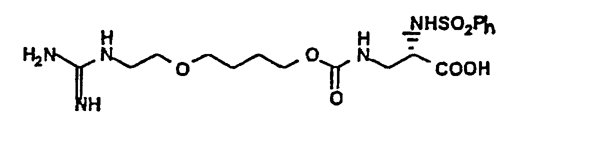

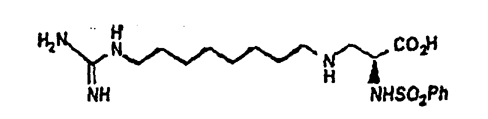

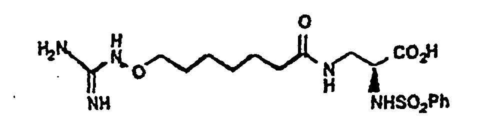

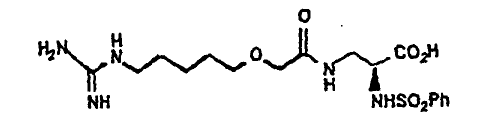

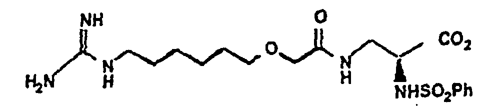

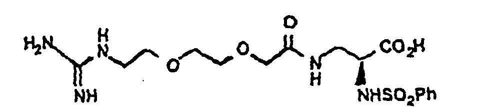

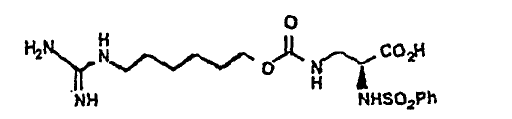

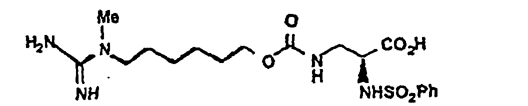

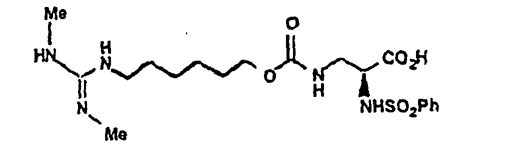

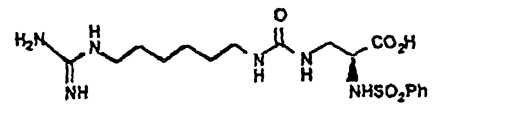

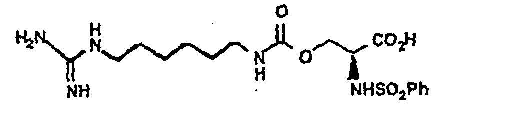

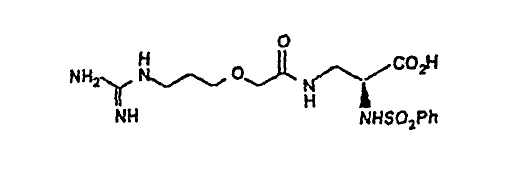

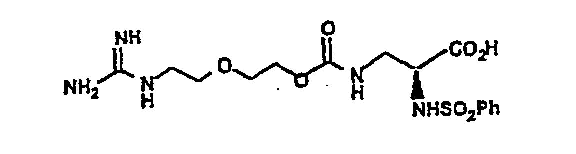

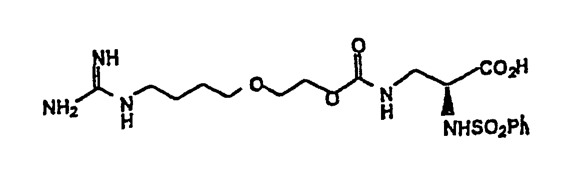

15. Composition pharmaceutique pour la prévention ou le traitement d'un état chez un mammifère, **caractérisé par** une thrombose indésirable, comprenant un support théraeutiquement acceptable et une quantité thérapeutiquement efficace d'un composé de formule I.

16. Utilisation d'un composé selon la revendication 1, pour la fabrication d'un médicament pour la prévention
ou le traitement d'un état chez un mammifère **caractérisé par** une thrombose indésirable.

17. Utilisation selon la revendication 16, dans lequel l'état est sélectionné dans le groupe consistant en : le syndrome coronaire aigu, l'infarctus du myocarde, l'angor instable, l'angor réfractaire, la thrombose coronaire occlusive se produisant après une thérapie thrombolytique ou après une angioplastie coronaire, un syndrome cérébro-vasculaire induit thrombolytiquement, une attaque embolique, une attaque thrombotique, des attaques ischémiques transitoires, une thrombose veineuse profonde, une embolie pulmonaire, une coagulopathie, une coagulation intravasculaire disséminée, le purpura thrombocytopénique thrombotique, la thromboangéite oblitérante, une maladie thrombotique associée à une thrombocytopénie induite par l'héparine, des complications thrombotiques associées à la circulation extracorporelle, des complications thromboiques associées à l'instrumentation telle qu'une cathétérisation cardiaque ou intravasculaire autre, une pompe de ballonnet intra-aortique, un stent coronaire ou une valve cardiaque, des états nécessitant l'adaptation de dispositifs de prothèse, la vascularisation de tumeurs solides et la rétinopathie.

18. Procédé de fabrication d'un amide lié à un éther ayant la formule générale :
Z-(L)-Q₁
dans laquelle Q₁ représente 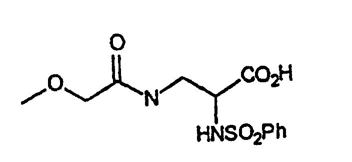
Z est sélectionné dans le groupe consistant en -NH-C(R⁹)=NR¹¹, -C(NR⁹R¹⁰)=NR¹¹ et pipéridinyle ; où R⁹, R¹⁰ et R¹¹ sont indépendamment sélectionnés dans le groupe consistant en H, alkyle en C₁₋₆, aryl-alkyle en C₁₋₃ et aryle ; ou bien où deux des radicaux R⁹, R¹⁰ ou R¹¹ forment un noyau cyclique contenant (CH₂)ₚ, où p = 2-5 ;
L est un groupe de liaison sélectionné dans le groupe consistant en une liaison, -O-, un groupe bivalent dérivant d'un alkane en C₁₋C₈, ou un groupe alkoxy bivalent de formule générale -O-C₁-C₈
lequel procédé comprend les étapes successives suivantes :
(a) la réaction d'un amino-alcool N-protégé en présence d'une quantité catalytique d'un catalyseur au rhodium avec un diazoacétate d'alkyle pour donner un alpha alkoxy éther ;
(b) la saponification du produit issu de la réaction de l'étape (a) avec un hydroxyde métallique pour donner un acide carboxylique ;
(c) la réaction du produit de l'étape (b) avec une amine et avec un agent de déshydratation carbodiamide pour donner ledit amide lié à un éther.

19. Procédé de fabrication d'un carbamate lié à un éther ayant la formule générale
Z-(L)-Q₂
dans laquelle
Q₂ représente 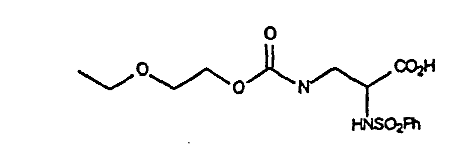
Z est sélectionné dans le groupe consistant en -NH-C(R⁹)=NR¹¹, -C (NR₉R₁₀) -NR¹¹ et pipéridinyle ; où R⁹, R¹⁰ et R¹¹ sont indépendamment sélectionnés dans le groupe consistant en H, alkyle en C₁₋₆, aryl-alkyle en C₁₋₃ et aryle ; ou bien où deux des radicaux R⁹, R¹⁰ ou R¹¹ forment un noyau cyclique contenant (CH₂)ₚ, où p = 2-5 ;
L est un groupe de liaison sélectionné dans le groupe consistant en une liaison, -O-, un groupe bivalent dérivant d'un alkane en C₁₋C₈, ou un groupe alkoxy bivalent de formule générale -O-C₁-C₈- ;
lequel procédé comprend les étapes successives suivantes :
(a) la réaction d'un amino-alcool N-protégé en présence dune quantité catalytique d'un catalyseur au rhodium avec un diazoacétate d'alkyle pour donner un alpha alkoxy ester ;
(b) la saponification du produit issu de la réaction de l'étape (a) avec un hydroxyde métallique pour donner un acide carboxylique ;
(c) la réduction du produit issu de la réaction de l'étape (b) pour donner un alcool ; et
(d) la réaction du produit alcool issu de la réaction de l'étape (c) tout d'abord avec du phosgène puis avec une amine pour donner ledit carbamate lié à un éther.

20. Composé selon la revendication 1, pour une utilisation dans la prévention ou le traitement d'un état chez un mammifère **caractérisé par** une thrombose indésirable.

21. Composé selon la revendication 1, pour la prévention et le traitement d'un état sélectionné dans le groupe consistant en : le syndrome coronaire aigu, l'infarctus du myocarde, l'angor instable, l'angor réfractaire, la thrombose coronaire occlusive se produisant après une thérapie thrombolytique ou après une angioplastie coronaire, un syndrome cérébro-vasculaire induit thrombolytiquement, une attaque embolique, une attaque thrombotique, des attaques ischémiques transitoires, une thrombose veineuse profonde, une embolie pulmonaire, une coagulopathie, une coagulation intravasculaire disséminée, le purpura thrombocytopénique thrombotique, la thromboangéite oblitérante, une maladie thrombotique associée à une thrombocytopénie induite par l'héparine, des complications thrombotiques associées à la circulation extracorporelle, des complications thrombotiques associées à l'instrumentation telle qu'une cathétérisation cardiaque ou intravasculaire autre, une pompe de ballonnet intra-aortique, un stent coronaire ou une valve cardiaque, des états nécessitant l'adaptation de dispositifs de prothèse, la vascularisation de tumeurs solides et la rétinopathie.
